# EUROPEAN PATENT APPLICATION

(11) **EP 3 088 387 A1**
(43) Date of publication of application: **02.11.2016**
(21) Application number: 15165164.3
(22) Date of filing: 27.04.2015
(51) Int. Cl.: C07C 303/40, C07C 307/02, C07C 213/02, C07C 215/42

(54) **1,2-DISUBSTITUTED DIAMONDOIDS, METHOD FOR SYNTHESIZING 1,2-DISUBSTITUTED DIAMONDOIDS AND USAGE OF 1,2-DISUBSTITUTED DIAMONDOIDS**

(71) Applicant: Justus-Liebig-Universität Gießen, 35390 Gießen (DE)
(72) Inventor: Schreiner, Peter R., 35435 Wettenberg (DE); Hrdina, Radim, 35392 Gießen (DE)
(74) Representative: Stumpf, Peter

(57) **Abstract**

The invention comprises a straightforward way to 1,2-disubstituted diamondoids utilizing Rhcatalyzed (1 mol%) nitrenoid C-H bond amination, the 1,2-disubstituted diamondoids themselves and their medical as well as synthetic use as building blocks for further substances of diamondoid structure.

## Description

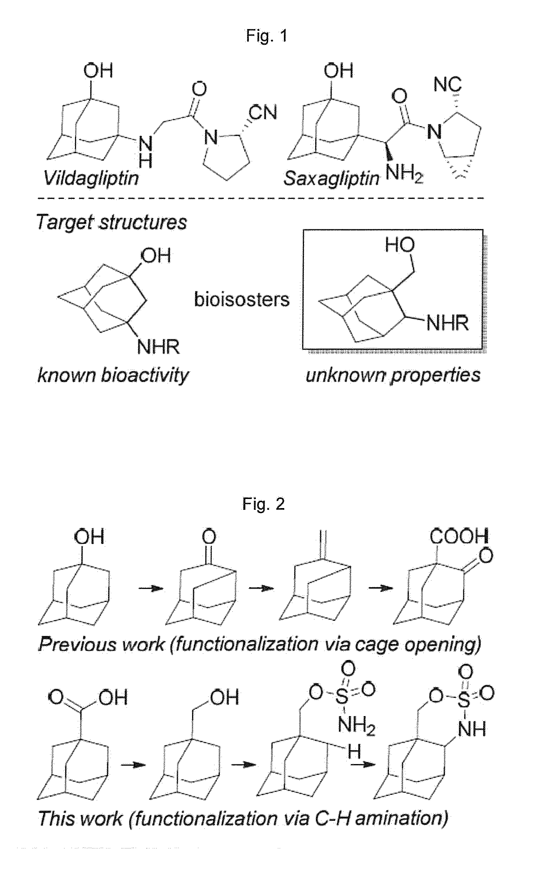

Owing to the highly lipophilic but otherwise chemically inert character of the adamantane moiety, which has been incorporated in number of bioactive compounds and commercial drugs, there is a general quest for new syntheses of diamondoid-based derivatives. In this work we focus on C-H amination reactions and present a synthesis of the bioisosters of the antihyperglycemic agent DPP-4 inhibitor "Vildagliptin^{®}" (Fig 1).

Generally, the selective C-H functionalization of diamondoids can be divided into two categories: *undirected* functionalizations such as oxidation reactions, where the selectivity is based on the different C-H dissociation energies or thermodynamic stabilities of corresponding ions or radicals; these lead in most cases to tertiary products. *Directed* functionalizations using intramolecular reactions to obtain 1,2-disubstituted diamondoids are rather rare. Here we present a new approach to disubstituted diamondoids through rhodium (II) catalyzed intramolecular C-H nitrene insertion reactions of sulfamate esters (Fig. 2). This approach was chosen for two reasons: First, the high reactivity of metal-bound nitrene species would allow functionalization of unactivated rigid diamondoids. Second, the regioselectivity of nitrene insertions towards the formation of six-membered rings. The ultimate goal was to find an approach to β-substituted diamondoid derivatives without cage opening, which is currently the standard path to the 1,2-disubsitution pattern.

Among published protocols, the C-H amination reaction of sulfamates developed by the Du Bois group seemed to be most appropriate to reach the target structures. Retrosynthetically, the starting materials are accessible in only two steps from readily available carboxylic acids, and the key reaction, nitrenoid insertion reaction, is typically effectively catalyzed by low loadings of transition metal catalysts (Rh, Ag, Fe, Ru, Cu, Co/Pd, and Mn). The products of the insertion reactions, cyclic sulfamates, are universal starting materials for further transformations. Their oxidation to the corresponding imines opens an avenue to keto- or tetrasubstituted carbon derivatives. The reductive cleavage is a direct way to 1,3-amino alcohols, precursors of β-amino acids, catalysts, ligands, and a variety of bioactive compounds.

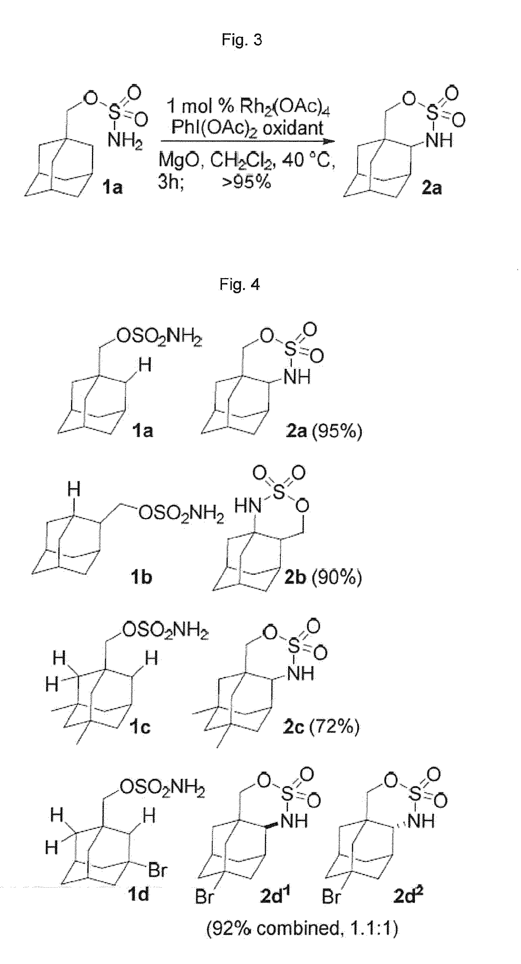

Reduction of adamantane-1-carboxylic acid with LiAlH₄ and subsequent esterification with sulfamoyl chloride provided ready access to starting material 1a (Fig. 3). It is obvious to the person skilled in the art, that other suitable substances which provide effective leaving groups for the ring-closure reaction may also be used. The list of suitable substances contains for example but not exclusively substances of the general formula Ri-OSO₂N₃, R₁-OSONH₂, R₁-OSO₂NHR₂ and R₁-OSONHR₂, where R₁ and R₂ are independently from each other be chosen from the following list, containing for example but not exclusively halogenid-, tosyl-, mesyl- and CF₃COO-.

This dynamically C₃-symmetric monosubstituted adamantane derivative displays six equivalent C-H bonds in the neighboring position of the directing group that are prone to undergo C-H insertion. Rh₂(OAc)₄ was chosen as the transition metal catalyst, phenyliodoacetate as oxidant, MgO as base and dichloromethane as solvent for the test reactions. It is obvious to the person skilled in the art that the invention is not confined to this specific combination of catalyst, oxidant, base and solvent to achieve this reactive step of the synthesizing method for 1,2-disubstituted diamondoids. It is possible to use, for example, PhlO instead of Phl(OAcyl)₂ as oxidation agent, CHCl₃ or other similar organic solvents instead of CH₂Cl₂ as solvent and other suitable metal oxides instead of MgO as base without leaving the scope of the invention or its equivalents.

To our delight the reaction proceeded quantitatively with 2 mol% of the catalyst. The catalyst loading can be reduced to 1 mol% and works well on gram scale (up to 4 g of **1a** were employed). These conditions were then used as our standard for other derivatives to determine the scope of this transformation (Fig. 4). It is obvious to the person skilled in the art that the invention is not confined to the synthesis of the derivatives shown in Fig. 4.

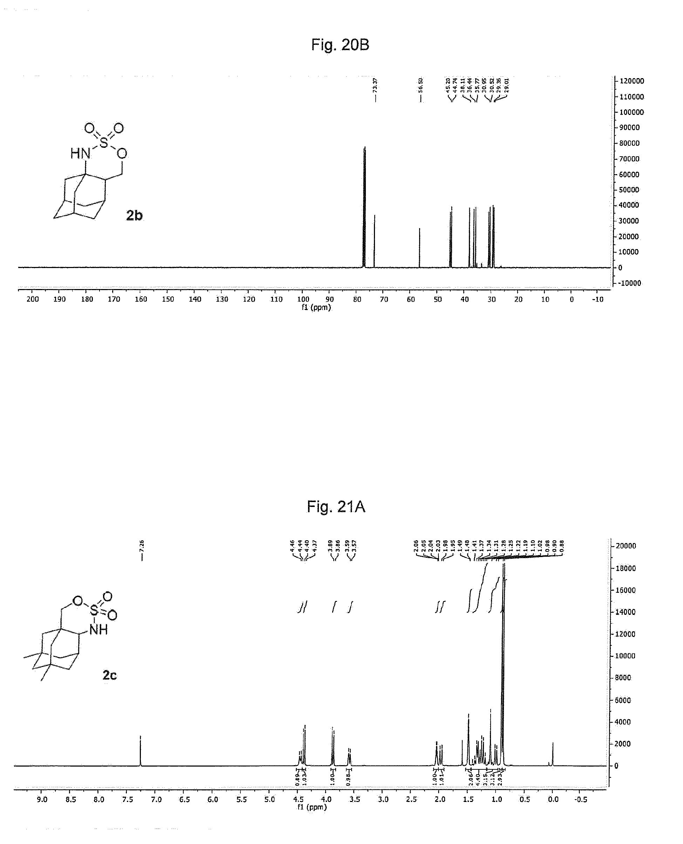

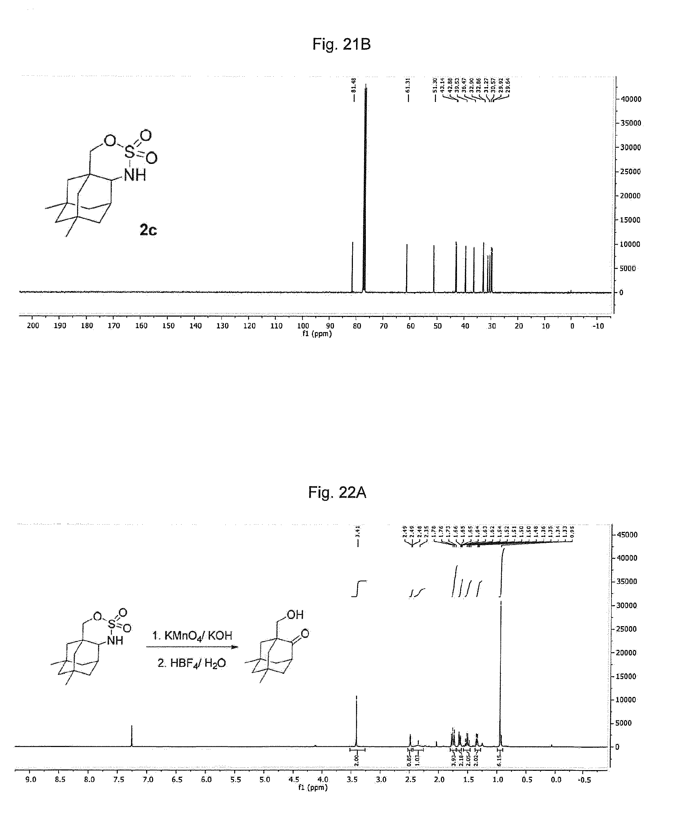

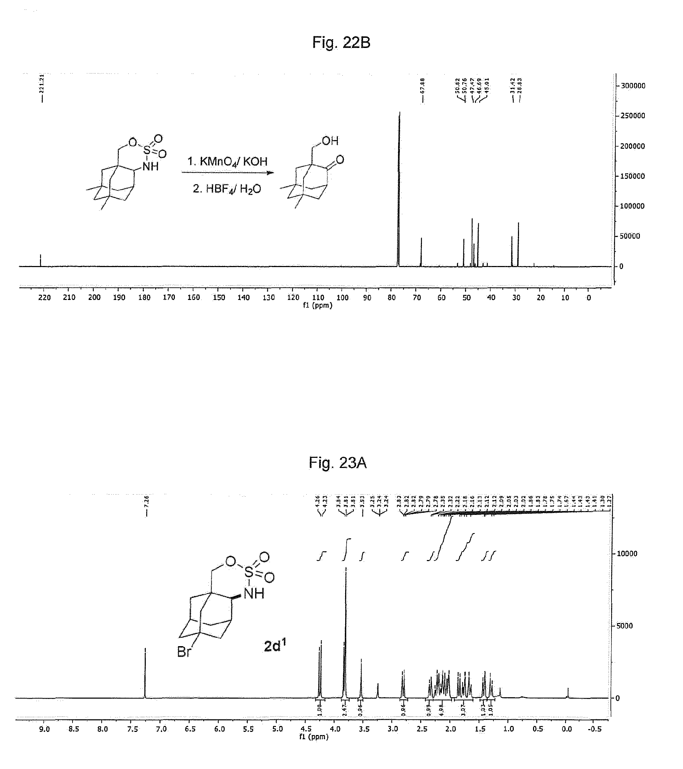

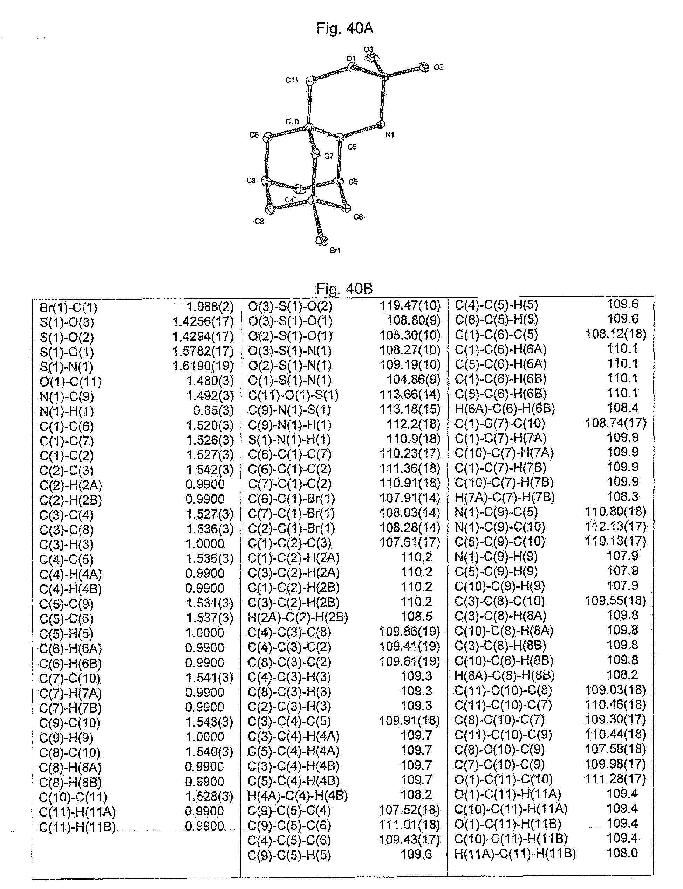

Next, the insertion reactions were performed with **1b**-**h**, all of which display different C-H bond environments. The regioselectivity of the insertion reaction was tested with dimethyl-substituted 1c and bromo-substituted 1d. For **1c** the insertion reaction proceeded quantitatively with 83% regioselectivity in favor of the activated C-H bonds that hyperconjugate with the substituents. Steric effects can be excluded as the methyl groups are far away from the reaction center. Simple crystallization from ethylacetate provided 72% of 2c (structure confirmed by sequence of reactions to symmetrical 2-oxo derivative; see corresponding example and Fig. 21A / Fig. 21B / Fig. 22A / Fig. 22B). In the case of 2d the insertion reaction provided corresponding epimers in 92% yield. Epimeric products **2d¹** and **2d²** were isolated in a 1.1:1 ratio; isomer **2d¹** was crystallized and its structure confirmed by X-ray diffraction analysis (Fig. 40A).

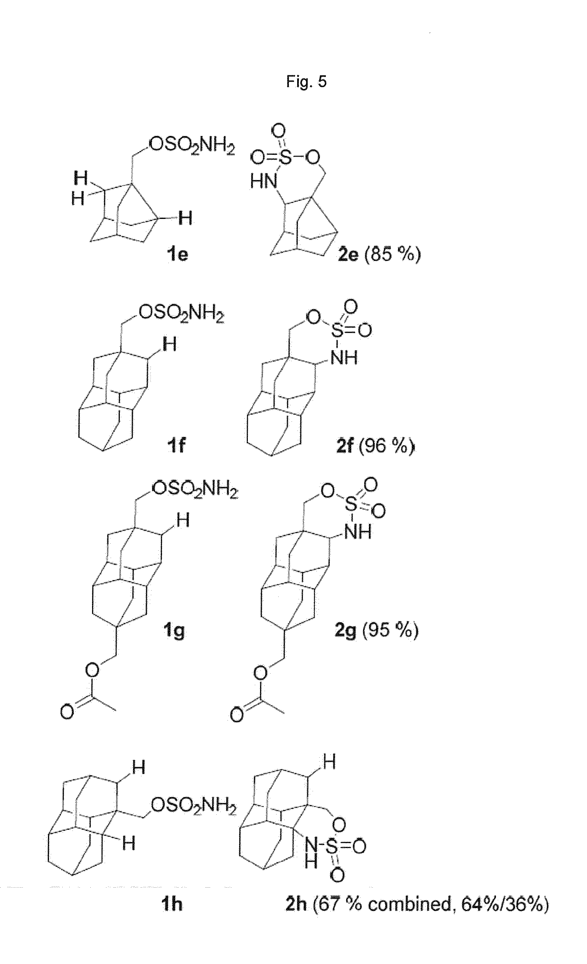

Then we focused on derivatives other than adamantane (Fig. 5). We began with noradamantane derivative 1e, which has three different C-H bonds in the neighboring position. The insertion reaction produced exclusively 2e. Due to the strain in the noradamantane cage, the formal sp³ hybridization of the tertiary C-H bond is reduced (i.e., larger s-content) and the C-H bond dissociation energy as compared to the secondary position is higher. As a consequence, the secondary position is favored and exo-hydrogen of the diastereotopic pair is geometrically more accessible. Diamantane derivatives **1f** and **1 g** are the next higher diamondoid congeners and the insertion reaction proceeds equally well in both cases. Derivative **1 h** with the functional group in the medial position has two sets of different C-H bonds. As tertiary C-H bonds are more reactive towards the insertion reactions versus secondary ones, the ratio between the two regioisomers was 64:36 in favor of bis-medial substitution. The structure of the major isomer was confirmed using X-ray diffraction analysis. It is obvious to the person skilled in the art that the invention is not confined to the synthesis of the derivatives shown in Fig. 4 and Fig. 5, but that it can be employed to numerous other substances with adamantane-like and diamantane-like cage-structures.

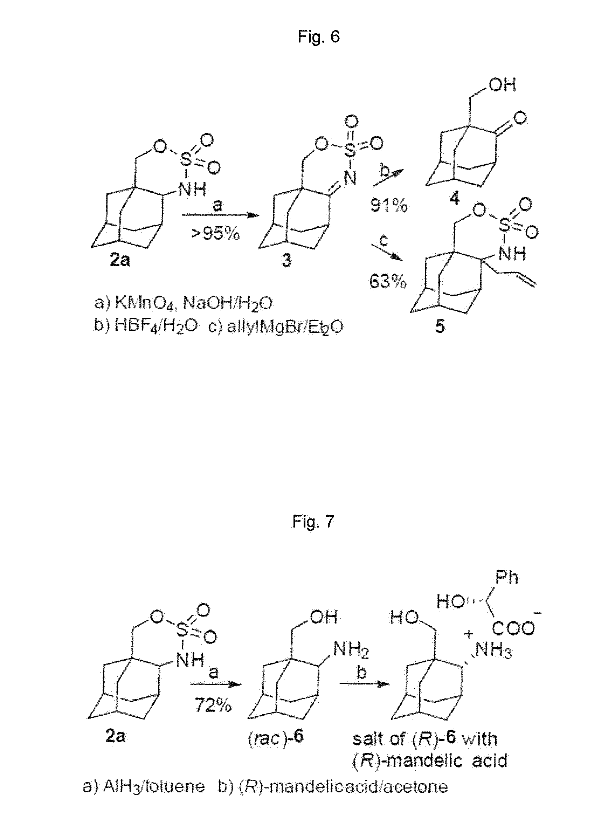

To demonstrate the utility of the method we chose **2a** for further chemical transformations. The permanganate oxidation of the sulfamidate moiety in basic medium to the corresponding imine 3 went smoothly. The person skilled in the art knows that there are other well known methods available with which one can achieve the oxidation of **2a** to **3.** Any such other method for oxidation **of 2a** to **3** is within the scope of the invention and its equivalents. Using non-coordinating acid HBF₄, imine **3** was hydrolyzed to compound **4,** a common intermediate in organic synthesis. The reactivity of **3** towards nucleophilic addition was tested on the reaction with allyl magnesium bromide, demonstrating access to quaternary substitution on the rigid adamantane skeleton (Fig. 6). It is obvious to the person skilled in the art that the invention is not confined to these two specific further chemical transformations of 2a. Any other chemical transformation that can be applied to **2a** without cleaving the adamantane core structure falls within the scope of the invention and its equivalents.

Deprotection of the cyclic sulfamidate moiety under strong acidic or basic conditions failed. Another possibility to obtain the corresponding amino alcohols is reductive cleavage. The reducing agent of choice was NMeEt₂*AlH₃, a complex of AlH₃ and the tertiary amine NMeEt₂, which gave **6** in 72% yield. It is obvious to the person skilled in the art that the invention is not confined to this specific choice of reducing agent to achieve this reactive step of the synthesizing method for 1,2-disubstituted diamondoids. The usage of many other reducing agents, formed by combination of, e.g. LiALH₄, NaH etc., with other amines also falls within the scope of the invention and its equivalents. For the resolution of the racemic mixture of 6, enantiopure mandelic acid was found to be a very good resolution agent. Salt formation with R-mandelic acid proceeded quantitatively and diastereomeric salt of **(-)-(6)** crystallized preferentially from acetone (Fig. 7). It is obvious to the person skilled in the art that the invention is not confined to this specific choice of resolution agent to achieve the separation of the two enantiomeric products of this step of the synthesizing method for 1,2-disubstituted diamondoids. The usage of any other resolution agent, well known in the state of the art, for example lactic acid, also falls within the scope of the invention and its equivalents.

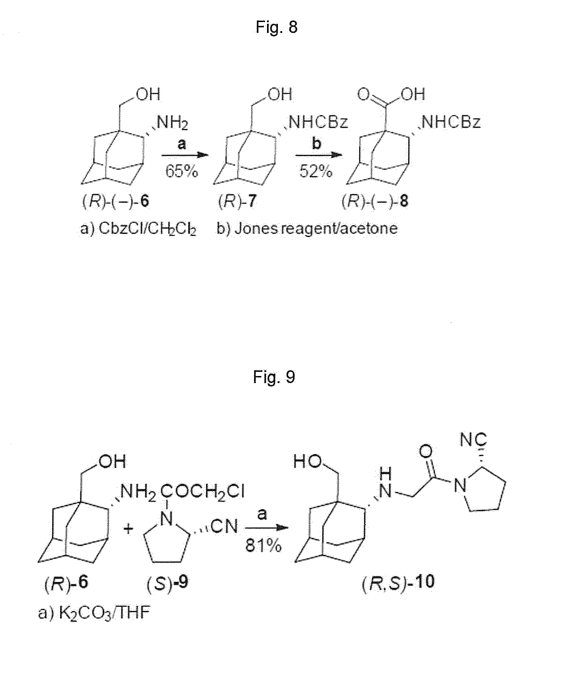

The absolute configuration of (-)-**6** was determined using diffraction analysis of a single crystal of the (-)-**6** and (R)-mandelic acid salt. Both diastereomeric salts were separated and corresponding amine was released using potassium carbonate. Both enantiomers were then recrystallized separately with corresponding enantiomers of mandelic acid to obtain high en-antipurity of **6.** Upon the second hydrolysis using the potassium carbonate both pure enantiomers of **6** were used for further synthesis. Enantiopure (*R*)-(-)-**6** was used for further derivatizations. Cbz protection of the amine group proceeded in 65% yield. It is well known to the person skilled in the art, that there are numerous other substances available for protecting amine groups. They can also be used within the scope of the invention and its equivalents. Compound (R)-7 was then oxidized to a carboxylic acid (R)-(-)-8 with Jones reagent in 52% yield (Fig. 8). It is well known to the person skilled in the art, that there are numerous other substances/mixtures available for oxidizing hydroxyl groups to carboxylic groups. They can also be used within the scope of the invention and its equivalents.

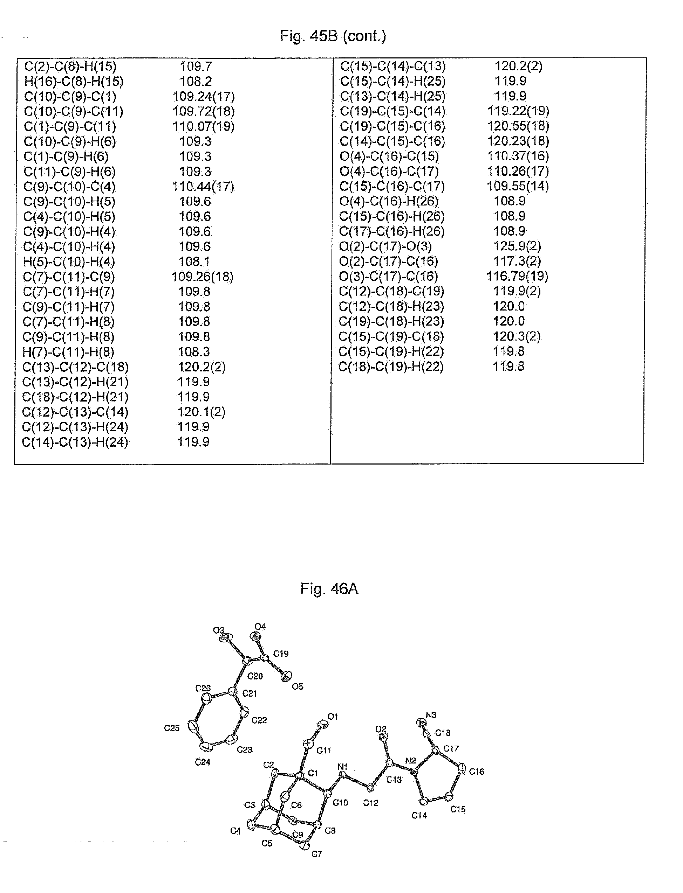

Finally, both enantiomers of **6** were used for the preparation of the targeted structural analogs of Vildagliptin^{®}. Condensation of (*R*) resp. (*S*)-**6** with pyrrolidine derivative (*S*)-**9** provided the desired compounds in 81 (85)% yield (Fig. 9). To confirm the structure, compound (*R,S*)-**10** was crystallized as a salt with (*R*)-mandelic acid and analyzed by X-ray diffraction (Fig. 46A).

In summary, we have developed a straightforward way to 1,2-disubstituted diamondoids of general formula (I), general formula (II), general formula (III), general formula (IV), general formula (V), general formula (VI) and general formula (VII), utilizing Rh-catalyzed (1 mol%) nitrenoid C-H bond amination.

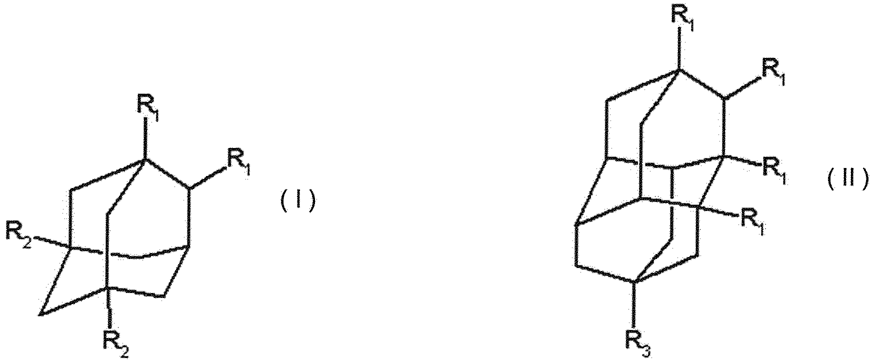

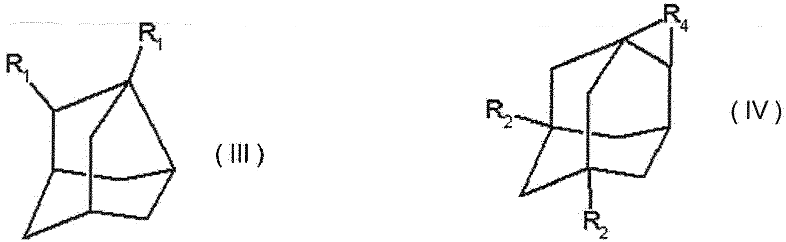

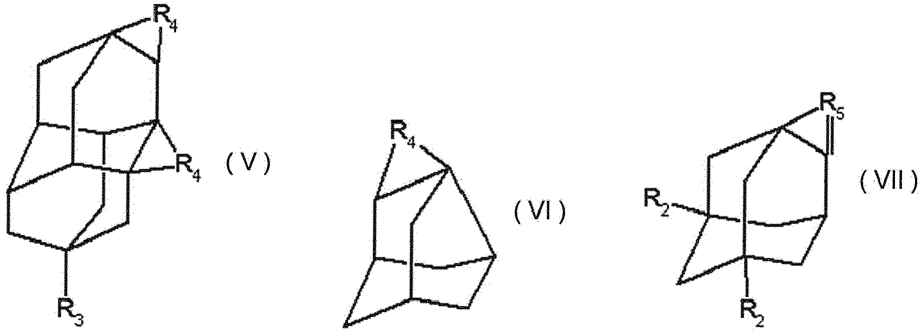

We applied this strategy to the synthesis of new analogs of Vildagliptin^{®}, which is an antidiabetic remedy. Testing of the biological properties of these compounds is in progress. The inventive adamantane-based substances may also be used for other medical applications, e.g. cytoprotection of lymphocytes, diseases in which cells are destroyed by cytopathic effects, infections of viruses, infections of retroviruses and borna disease virus infections.

Especially substance **6** is a very versatile usable substance: Due to its bi-functionality it can also be employed as monomer and co-monomer for polymers in order to modify the physical properties of the polymer. It can also be used as linker for cross-polymerisation of polymers. Because of the rigidity of the adamantoid core-structure it can also be used as linker for functionalizing surfaces, e.g. surfaces of nanoparticles, as well as using it as spacer within molecules used for molecular electronics. The same is true for substance **3,** which can also be subject to numerous chemical transformations, thus also leading to many different substances which can function as intermediate starting materials for further chemical synthesis.

Thus it is obvious to the person skilled in the art that the invention, revealed herein, is of great commercial applicability in many scientific fields.

It is obvious to the person skilled in the art, that the inventive method can be applied to numerous substances beyond the examples revealed herein. The scope of the invention and its equivalents is therefore in no way restricted to the examples shown herein. The invention comprises substances of the general formula (I), (II), (III), (IV), (V), (VI) and (VII), where:
R₁ is chosen from the list comprising -H, -CH₂-OSO₂NH₂, -CH₂-OSO₂N₃, -CH₂-OSONH₂,-CH₂-OSO₂NHR, -CH₂-OSONHR, and R is chosen from the list comprising halogenid-, tosyl-, mesyl-, CF₃COO-; R₂ is chosen from the list comprising -H, -CH₃, -CH₂CH₃, -Br, -Cl, -I; R₃ is chosen from the list comprising -H, CH₃-CO₂-CH₂-, C₂H₅-CO₂-CH₂-, C₃H₇-CO₂-CH₂-and C₄H₉-CO₂-CH₂-; R₄ is chosen from the list comprising -CH₂-OSO₂NH-, -CH₂-OSONH-and R₅ is chosen from the list comprising -CH₂-OSO₂N=, -CH₂-OSON= in such a way that one of two R₁-substiuents at neighboring C-atom positions of the cage structures consists of just one H-atom and the second R₁-substituent is any other one substituent of the possible R₁-Substituents.

The invention further comprises:
Substances of the general structure as described before which are deprotonated by use of an organic or inorganic base and thus are available as salts and Substances of the general structure as described before which are protonated by use of an organic or inorganic acid and thus are available as salts.

The invention further comprises a method for synthesizing 1,2-disubstituted diamondoids of the general formula (IV), (V) and (VI) characterized in that the method comprises the following steps:
1. esterification of hydroxymethyl diamondoid by use of a substance chosen from the list comprising R₆-SO₂N₃, R₆-SONH₂, R₆-SO₂NHR₇, R₆-SONHR₇, where R₆ and R₇ are independently from each other be chosen from the list, comprising halogenid-, tosyl-, mesyl- and CF₃COO-,
2. performing on the product of step 1 a transition metal catalysed intramolecular oxidative insertion reaction of the functional group introduced via step 1 into a neighboring CH-bond of the diamondoid core structure.

The invention further comprises a method for synthesizing 1,2-disubstituted diamondoids according to the aforementioned method, leading to diamandoids of the general formula (I ), (II) and (III), characterized in that the product of step 2 from the aforementioned method is further processed by the following steps:
3. performing on the product of step 2 a reductive cleavage of the cyclic sulfa-mate N-S bond and/or O-S bond by usage of a reducing agent chosen from the list comprising NMeEt₂*AlH₃ complex, AlH₃, LiAlH₄ and NaH, leading to a racemic mixture of products,
4. separating the racemic mixture of products from step 3 into the enantiomeric components via salt formation with enantiopure mandelic acid and recrystallisation the mixture of salts,
5. releasing the free enantiopure 1,3-amino-alcohols from the products of step 4 by use of organic or inorganic base chosen from the list comprising carbonates, hydroxides, hydrogencarbonates, and
6. condensation of each of the enantiopure products of step 5 with pyrrolidine derivate 9.

The invention further comprises a Method for synthesizing 1,2-disubstituted diamondoids according to the aforementioned method, leading to diamondoids of the general formula ( VII), characterized in that the product of step 2 from the aforementioned method is further processed by the following steps:
7. oxidizing the product of step 2 by use of alkaline aqueous permanganate solution and
8. further functionalizing the product from step 7 by usage of a method chosen from the list comprising the methods hydrolysation with Broensted acids and nucleophilic addition with Grignard-reagent.

The invention further comprises the following applications of the inventive substances according to the general formula (I), (II), (III), (IV), (V), (VI) and (VII):
Use of the inventive substances as pharmaceutical component for medical treatment of mammalian diseases, characterized in that the disease to be treated is chosen from the list comprising autoimmune diseases, neurodegenerative diseases, diseases in which cells are destroyed by cytopathic effects, infections of viruses, infections of retroviruses and borna disease virus infections.

Use of at least one inventive substance as monomer and/or co-monomer for polymerization.

Use of at least one inventive substance as polymer-additive.

Use of at least one inventive substance as polymer-additive for crosslinking polymers.

Use of at least one inventive substance for functionalizing surfaces of macroscopic, microscopic or nanoscale objects.

Use of the inventive substances as intermediates/building blocks in the synthesis of bioactive compounds, ligands for metals, organocatalysts, surfactants, isolants, and macrocycles.

It is obvious to the person skilled in the art, that the invention is also comprising the salts of the deprotonated anionic forms of the inventive substances with organic or inorganic cations as well as the salts of the protonated cationic forms of the inventive substances with organic or inorganic anions.

### Description of the drawings

Fig. 1. Adamantane derived amino alcohols: Vildagliptin® and Saxagliptin® (commercial DPP-4 inhibitors)
Fig. 2. Novel route to 1,2-disubstituted diamondoids utilizing C-H amination as the key reaction
Fig. 3. Dirhodium acetate catalyzed intramolecular C-H bond nitrene insertion in 1a
Fig. 4. Intramolecular C-H amination of adamantane derivatives with depicted regioselectivity, yield of isolated isomer, and X-ray structures of the products
Fig. 5. Intramolecular C-H amination of diamondoids with depicted regioselectivity, yield, and X-ray structures of the products
Fig. 6. Oxidation of the sulfamidate moiety and subsequent transformations
Fig. 7. Reductive deprotection of the sulfamidate moiety and enantiomeric resolution
Fig. 8. Synthesis of enantiopure N-protected β-amino acid
Fig. 9. Synthesis of Vildagliptin® analogue
Fig. 10. Structural analogs of Vildagliptin®
Fig. 11A: ¹H-NMR-data of substance **1a**
Fig. 11B: ¹³C-NMR-data of substance **1a**
Fig. 12A: ¹H-NMR-data of substance **1b**
Fig. 12B: ¹³C-NMR-data of substance **1b**
Fig. 13A: ¹H-NMR-data of substance **1c**
Fig. 13B: ¹³C-NMR-data of substance **1c**
Fig. 14A: ¹H-NMR-data of substance **1d**
Fig. 14B: ¹³C-NMR-data of substance **1d**
Fig. 15A: ¹H-NMR-data of substance **1e**
Fig. 15B: ¹³C-NMR-data of substance **1e**
Fig. 16A: ¹H-NMR-data of substance **1f**
Fig. 16B: ¹³C-NMR-data of substance **1f**
Fig. 17A: ¹H-NMR-data of substance **1g**
Fig. 17B: ¹³C-NMR-data of substance **1g**
Fig. 18A: ¹H-NMR-data of substance **1h**
Fig. 18B: ¹³C-NMR-data of substance **1h**
Fig. 19A: ¹H-NMR-data of substance **2a**
Fig. 19B: ¹³C-NMR-data of substance **2a**
Fig. 20A: ¹H-NMR-data of substance **2b**
Fig. 20B: ¹³C-NMR-data of substance **2b**
Fig. 21A: ¹H-NMR-data of substance **2c**
Fig. 21 B: ¹³C-NMR-data of substance **2c**
Fig. 22A: ¹H-NMR-data of oxidation product of substance **2c**
Fig. 22B: ¹³C-NMR-data of oxidation product of substance **2c**
Fig. 23A: ¹H-NMR-data of substance **2d¹**
Fig. 23B: ¹³C-NMR-data of substance **2d¹**
Fig. 24A: ¹H-NMR-data of substance **2d²**
Fig. 24B: ¹³C-NMR-data of substance **2d²**
Fig. 25A: ¹H-NMR-data of substance **2e**
Fig. 25B: ¹³C-NMR-data of substance **2e**
Fig. 26A: ¹H-NMR-data of substance **2f**
Fig. 26B: ¹³C-NMR-data of substance **2f**
Fig. 27A: ¹H-NMR-data of substance **2g**
Fig. 27B: ¹³C-NMR-data of substance **2g**
Fig. 28A: ¹H-NMR-data of substance **2h¹**
Fig. 28B: ¹³C-NMR-data of substance **2h¹**
Fig. 28C: Reaction scheme for synthesizing substances **2h¹** and **2h²**
Fig. 29A: ¹H-NMR-data of substance **3**
Fig. 29B: ¹³C-NMR-data of substance **3**
Fig. 30A: ¹H-NMR-data of substance **6**
Fig. 30B: ¹³C-NMR-data of substance **6**
Fig. 31A: ¹H-NMR-data of substance **4**
Fig. 31 B: ¹³C-NMR-data of substance **4**
Fig. 32A: ¹H-NMR-data of substance **5**
Fig. 32B: ¹³C-NMR-data of substance **5**
Fig. 33A: ¹H-NMR-data of substance **6**
Fig. 33B: ¹³C-NMR-data of substance **6**
Fig. 33C: Enantiomeric resolution of substance **6**
Fig. 34A: ¹H-NMR-data of substance **7**
Fig. 34B: ¹³C-NMR-data of substance **7**
Fig. 35A: ¹H-NMR-data of substance **8**
Fig. 35B: ¹H-NMR-data of salt of **8** with 1-phenylethyl amine
Fig. 35C: ¹³C-NMR-data of substance **8**
Fig. 35D: ¹³C-NMR-data of salt of **8** with 1-phenylethyl amine
Fig. 35E: HPLC-data of substance **8**
Fig. 36A: ¹H-NMR-data of salt of (R,S)-**10** and (R)-mandelic acid
Fig. 36B: ¹H-NMR-data of free Amine
Fig. 36C: ¹³C-NMR-data of salt of (R,S**)-10** with (R)-mandelic acid
Fig. 36D: ¹³C-NMR-data of free Amine
Fig. 37A: ¹H-NMR-data of salt of (S,S)-**10** and HCl
Fig. 37B: ¹H-NMR-data of free Amine
Fig. 37C: ¹³C-NMR-data of salt of (S,S)-**10** and HCl
Fig. 37D: ¹³C-NMR-data of free Amine
Fig. 38A: X-rax structure of substance **2a**
Fig. 38B: Table 1 - Bond lengths [Å] and angles [°] for substance **2a**
Fig. 39A: X-rax structure of substance **2b**
Fig. 39B: Table 2 - Bond lengths [Å] and angles [°] for substance **2b**
Fig. 40A: X-rax structure of substance **2d**
Fig. 40B: Table 3 - Bond lengths [Å] and angles [°] for substance **2d**
Fig. 41A: X-rax structure of substance **2e**
Fig. 41B: Table 4 - Bond lengths [Å] and angles [°] for substance **2e**
Fig. 42A: X-rax structure of substance **2f**
Fig. 42B: Table 5 - Bond lengths [Å] and angles [°] for substance **2f**
Fig. 43A: X-rax structure of substance **2g**
Fig. 43B: Table 6 - Bond lengths [Å] and angles [°] for substance **2g**
Fig. 44A: X-rax structure of substance **2h**
Fig. 44B: Table 7 - Bond lengths [Å] and angles [°] for substance **2h**
Fig. 45A: X-rax structure of (R)-6 with (R)-mandelic acid.
Fig. 45B: Table 8 - Bond lengths [Å] and angles [°] for (R)-6 with (R)-mandelic acid.
Fig. 46A: X-rax structure of (R,S)-10 as a salt with (R)-mandelic acid.
Fig. 46B: Table 9 - Bond lengths [Å] and angles [°] for (R,S)-10 as a salt with (R)-mandelic acid.

### Examples

¹H and ¹³C NMR spectra were recorded on Bruker AV600, AV400 or AV200 spectrometers, respectively, using TMS as the internal standard with chemical shifts given in ppm relative to TMS or the respective solvent residual peaks.

Infrared spectra were recorded on a Bruker IFS25 spectrometer.

MS / HRMS were recorded on a Finnigan MAT95 sectorfield spectrometer; ESI mass spectra were recorded on a Finnigan LCQDuo spectrometer using methanol solutions of the respective compounds. High resolution ESI mass spectrometry was performed on a MicroTOF (Bruker Daltonics) mass spectrometer using methanol solutions of the respective compounds.

Optical rotation was measured by using a Jasco P-2000 polarimeter.

HPLC separations were performed using Dionex P680 HPLC Pump equiped with RI Shodex RI-101 and UV Dionex UVD 170U detectors.

To the person skilled in the art, other commercially available laboratory devices are known, which can be used in an equivalent manner to acquire the inventive experimental data. Using different machinery is possible without leaving the scope of the invention or its equivalents.

The general measuring conditions were the following ones:
NMR spectra were recorded on 400 or 600 MHz machine at room temperature (25 °C) unless otherwise stated. ¹H-NMR chemical shifts are given in ppm relative to Me₄Si with the solvent resonance used as the internal standard (CDCl₃ δ = 7.26 ppm). ¹³C-NMR (101 or 151 MHz) chemical shifts were given in ppm relative to Me₄Si with the solvent resonance used as the internal standard (CDCl₃ = 77.16 ppm). IR spectra were recorded using an ATR sampler and are reported in wave numbers (cm⁻¹). Melting points (m.p.) were measured in open capillary tubes and were uncorrected. Optical rotations were measured in a thermostated (20 °C) 10.0 cm long microcell at 589 nm (Na) or 435 nm (Hg) and are reported as follows: [α]_{D} (c (g/mL), solvent). HPLC analyses were performed using analytical Chiralpak (0.46 cm x 25 cm) columns. Detection was performed using UV detector at 254 nm. Retention times (tᵣ) are given in minutes (min). All reactions involving air sensitive compounds were carried out under dry N₂ or argon by means of an inert gas/vacuum double manifold line and standard Schlenk techniques. Flash column chromatography was performed with silicagel 40-63.

In the following examples different types of solvents, drying agents (e.g. MgSO₄) etc. are revealed. It is obvious to the person skilled in the art, that other well known solvents, drying agents etc. with similar physical properties to the revealed ones may also be used without leaving the scope of the invention and its equivalents.

All temperature values mentioned in the following examples are meant to be understood as being the central value of a temperature range extending until +/- 10 °C, preferred until +/-5°C and most preferrend until +/- 2 °C of the revealed value.

All time specifications mentioned in the following examples are meant to be understood as being the central value of a time span ranging from +/- 30 % of the revealed time, preferred ranging from +/- 20 % of the revealed time and most preferred ranging from +/- 10 % of the revealed time.

### General synthesis of (1) esters of sulfamic acid and diamondoid derived alcohols:

Formic acid 325 µL was added to neat ClSO₂NCO 750 µL at 0 °C, after 10 minutes dichloromethane 4.5 mL was added and the reaction mixture was stirred at 0 °C for 1 h. After this time reaction mixture was heated to 25 °C and stirred for additional 8 h at this temperature.

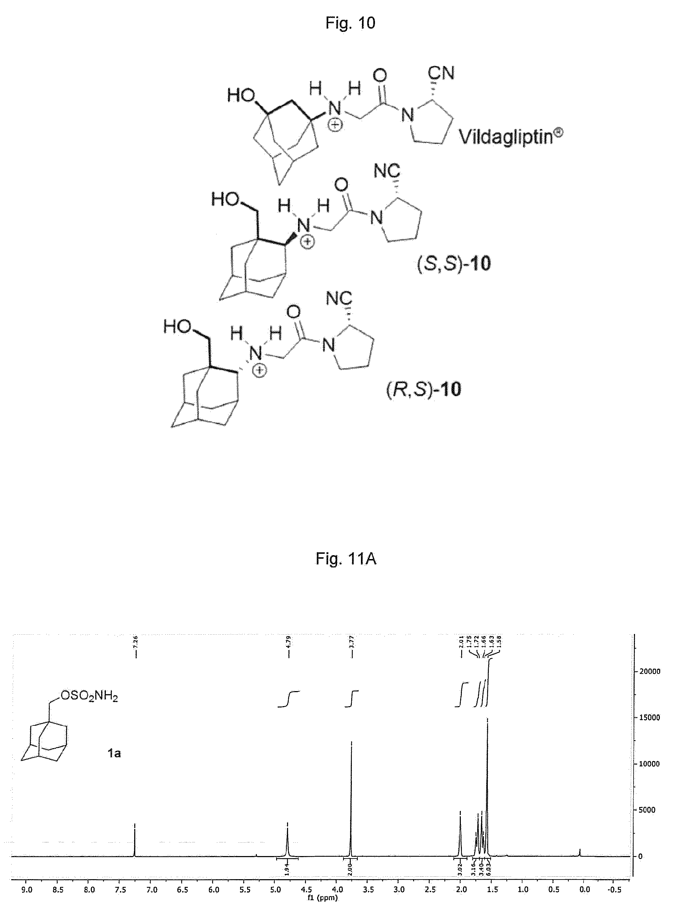

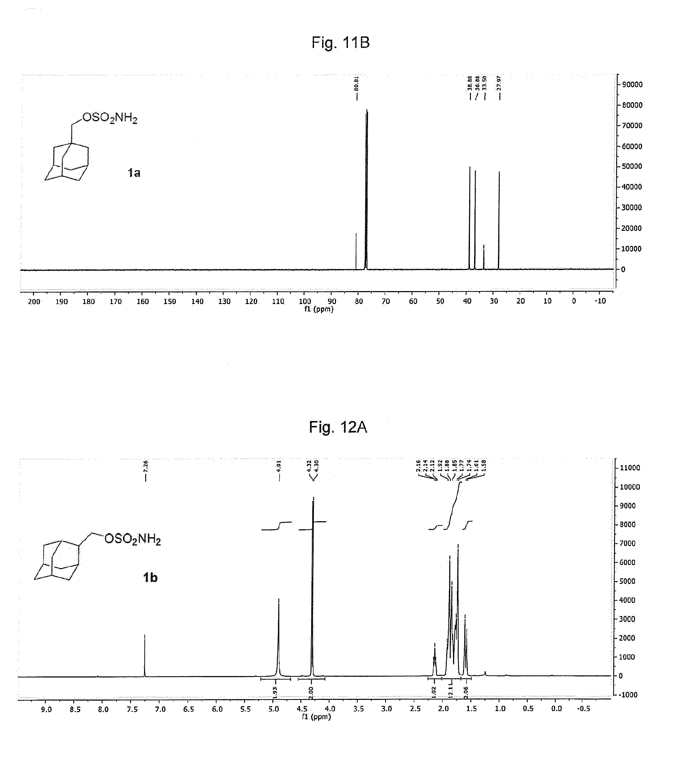

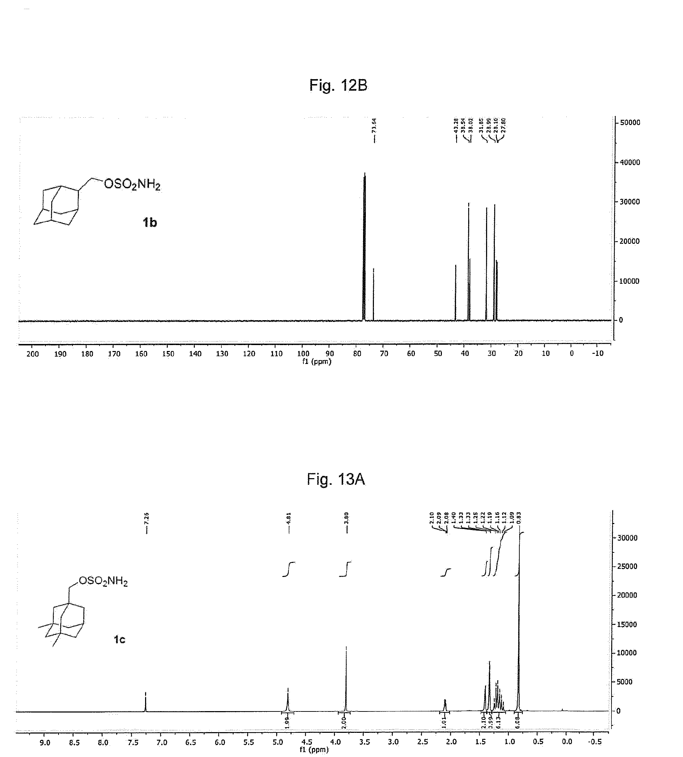

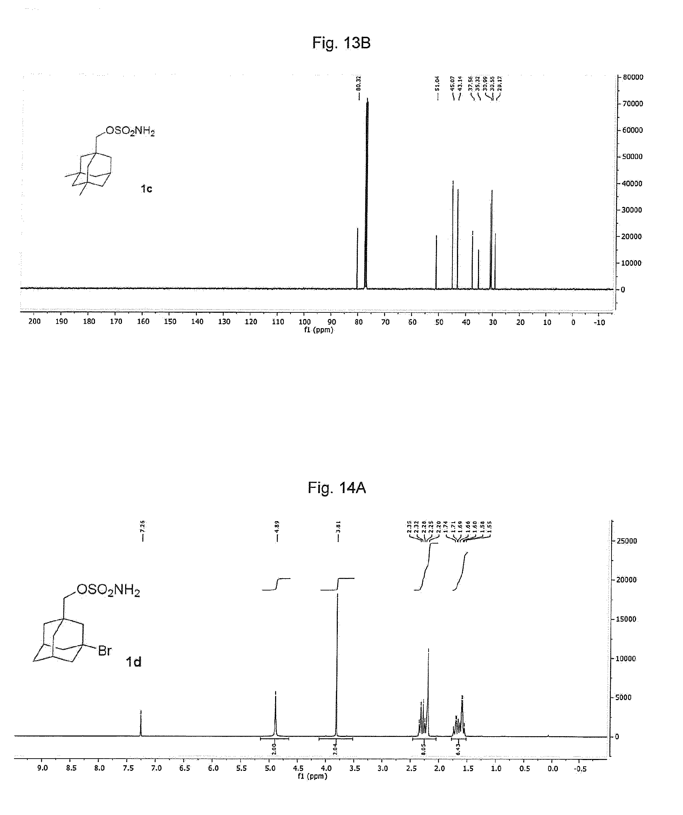

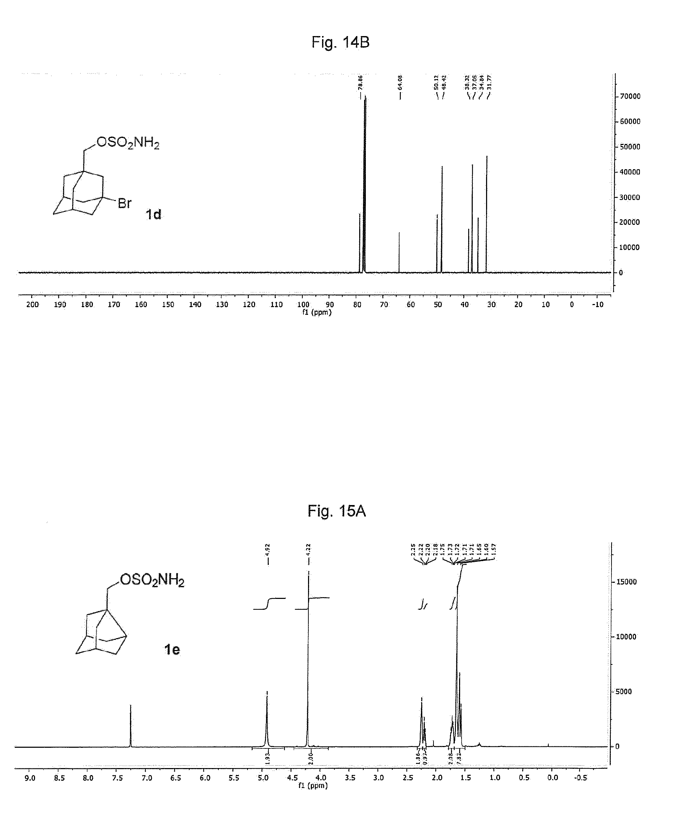

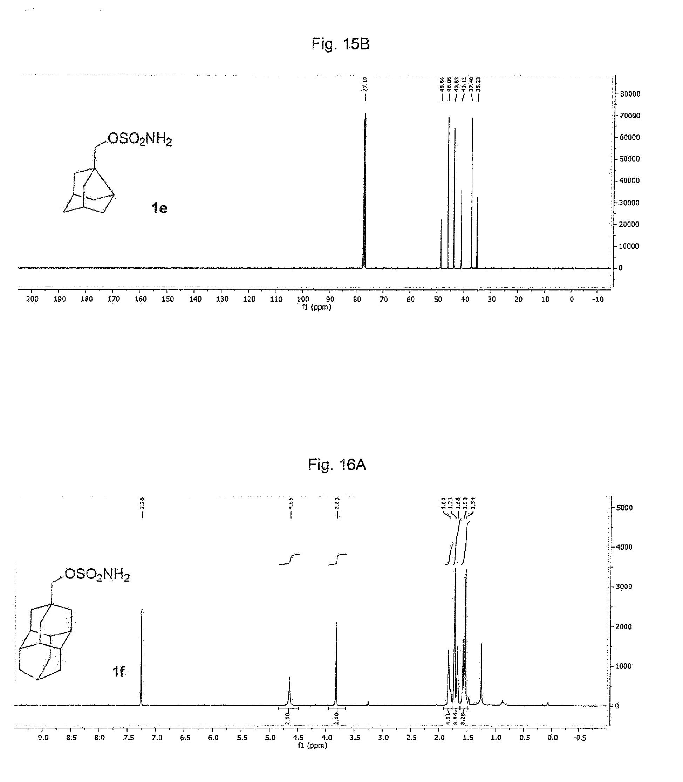

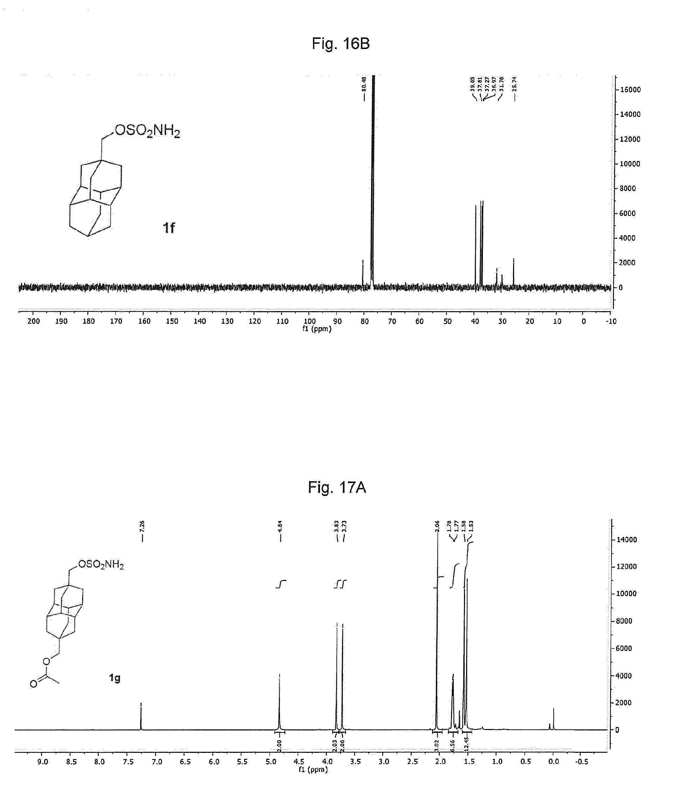

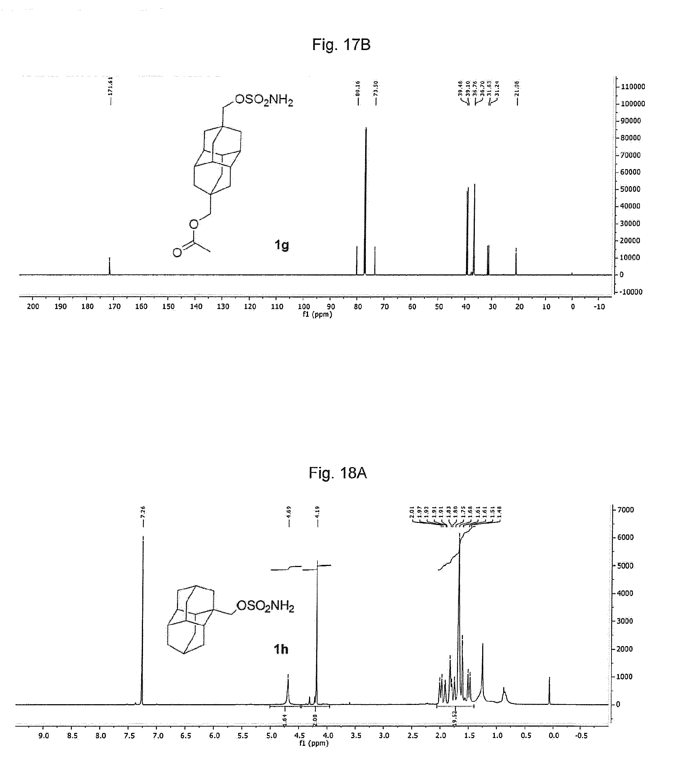

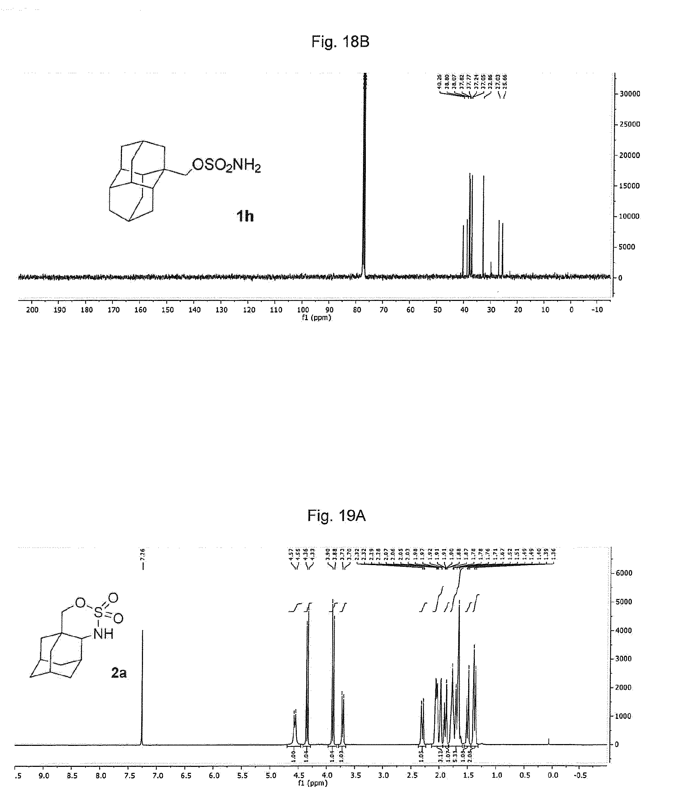

Then solution of 5.0 mmol of corresponding alcohol and 700 µL pyridine in 4.0 mL of dichloromethane was added dropwise at 0 °C and the reaction mixture was stirred for 12 h at 25 °C. After this time reaction was quenched with water and product was extracted using EtOAc. Organic fractions were collected, dried over MgSO₄, and solvent was then evaporated under vacuo. Column chromatography on silica gel using hexane/EtOAc mixture as mobile phase provided colorless solid product **1.**
physical data of substance **1a** (cf. Fig. 11A / Fig. 11 B):
   **Yield:** 1.13 g (90 %, 5.0 mmol scale)
   **R.f.** 0.33 (hexane/EtOAc 2/1, silica gel)
   **m.p.** 138.7 -139.4 °C (crystallized from EtOAc)
   **¹H NMR** (400 MHz, CDCl₃): δ/ppm = 1.58-1.63 (m, 6H), 1.63 -1.75 (m, 6H), 2.01 (bs, 3H), 3.77 (s, 2H), 4.79 (s, 2H).
   **¹³C NMR** (101 MHz, CDCl₃): δ/ppm = 28.0 (3CH), 33.5 (C), 36.9 (3CH₂), 38.9 (3CH₂), 80.8 (CH₂).
   **IR** (neat): ṽ/cm⁻¹ = 3343, 3258, 2905, 2849, 1577, 1461, 1359, 1184, 1160, 980, 964, 941, 919,850,808,727,681,563,546,514.
   **HRMS:** m/z = 245.10873 (calculated for C₁₁H₁₉NO₃S m/z = 245.10856)
physical data of substance **1b** (cf. Fig. 12A / Fig. 12B):
   **Yield:** 690 mg (78 %, 3.6 mmol scale)
   **R.f.** 0.40 (hexane/EtOAc 2/1, silica gel)
   **m.p.** 107.5-108.4 °C (crystallized from EtOAc)
   **¹H NMR** (400 MHz, CDCl₃): δ/ppm = 1.58 - 1.61 (m, 2H), 1.74 - 1.92 (m, 12H), 2.14 (t, *J* = 7.5 Hz, 1 H), 4.31 (d, *J* = 7.7 Hz, 2H), 4.91 (s, 2H).
   **¹³C NMR** (101 MHz, CDCl₃): δ/ppm = 27.8 (CH), 28.1 (CH), 29.0 (2CH), 31.8 (2CH₂), 38.0 (CH₂), 38.5 (2CH₂), 43.2 (CH), 73.6 (CH₂).
   **IR** (neat): ṽ/cm⁻¹ = 3375, 3280, 2915, 2852, 1561, 1454, 1359, 1170, 962, 927, 840, 799, 585,567,536.
   **HRMS:** m/z = 245.10817 (calculated for C₁₁H₁₉NO₃S m/z = 245.10856)
physical data of substance 1c (cf. Fig. 13A / Fig. 13B):
   **Yield:** 792 mg (73 %, 4.0 mmol scale)
   **R.f.** 0.40 (hexane/EtOAc 2/1, silica gel)
   **m.p.** 106.9 -107.4 °C (crystallized from EtOAc)
   **¹H NMR** (400 MHz, CDCl₃): δ/ppm = 0.83 (s, 6H), 1.09 - 1.25 (m, 6H), 1.33 - 1.34 (m, 4H), 1.39 - 1.41 (m, 2H), 2.08 - 2.10 (m, 1 H), 3.80 (s, 2H), 4.81 (s, 2H).
   **¹³C NMR** (101 MHz, CDCl₃): δ/ppm = 29.2 (CH), 30.6 (2CH₃), 31.0 (2C), 35.3 (C), 37.6 (CH₂), 43.1 (2CH₂), 45.1 (2CH₂), 51.0 (CH₂), 80.3 (CH₂).
   **IR** (neat): ṽ/cm⁻¹ = 3358, 3282, 2939, 2897, 2859, 2841, 1557, 1459, 1354, 1221, 1198, 989, 920, 842, 776, 640, 560.
   **HRMS:** m/z = 273.13929 (calculated for C₁₃H₂₃NO₃S m/z = 273.13986)
physical data of substance **1d** (cf. Fig. 14A / Fig. 14B):
   **Yield:** 1870 mg (90 %, 6.4 mmol scale)
   **R.f.** 0.25 (hexane/EtOAc 2/1, silica gel)
   **m.p.** 104.9 -105.1 °C (crystallized from EtOAc)
   **¹H NMR** (400 MHz, CDCl₃): δ/ppm = 1.55 - 1.74 (m, 6H), 2.20 - 2.35 (m, 8H), 3.81 (s, 2H), 4.89 (s, 2H).
   **¹³C NMR** (101 MHz, CDCl₃): δ/ppm = 31.8 (2CH), 34.8 (CH₂), 37.1 (2CH₂), 38.3 (C), 48.4 (2CH₂), 50.1 (CH₂), 64.1 (C), 78.9 (CH₂).
   **IR** (neat): ṽ/cm⁻¹ = 3354, 3326, 3262, 2932, 2910, 2856, 1569, 1460, 1362, 1306, 1284, 1163, 979, 959, 935, 853, 818, 743, 681, 564, 547, 516.
   **HRMS:** m/z = (M+Na⁺ , Maldi) 346.00820 (calculated for C₁₁H₁₈NBrO₃SNa m/z = 346.00830)
physical data of substance **1e** (cf. Fig. 15A / Fig. 15B):
   **Yield:** 290 mg (64 %, 2.0 mmol scale)
   **R.f.** 0.30 (hexane/EtOAc 2/1, silica gel)
   **m.p.** 97.3 - 98.1 °C (crystallized from EtOAc)
   **¹H NMR** (400 MHz, CDCl₃): δ/ppm = 1.58 - 1.75 (m, 10H), 2.14 - 2.26 (m, 3H), 4.22 (s, 2H), 4.86 (s, 2H).
   **¹³C NMR** (101 MHz, CDCl₃): δ/ppm = 35.3 (CH₂), 37.4 (2CH), 41.4 (CH), 43.8 (2CH₂), 46.1 (2CH₂), 48.7 (C), 77.2 (CH₂).
   **IR** (neat): ṽ/cm⁻¹ = 3330, 3249, 2921, 2866, 1575, 1459, 1359, 1163, 964, 845, 803, 693, 554, 535.
   **HRMS:** m/z = 231.09222 (calculated for C₁₀H₁₇NO₃S m/z = 231.09291)
physical data of substance **1f** (cf. Fig. 16A / Fig. 16B):
   **Yield:** 250 mg (84 %, 1.0 mmol scale)
   **R.f.** 0.30 (hexane/EtOAc 2/1, silica gel)
   **m.p.** 190.2 - 191.0 (crystallized from EtOAc)
   **¹H NMR** (400 MHz, CDCl₃): δ/ppm = 1.50 -1.73 (m, 15H), 1.75 -1.84 (m, 4H), 3.83 (s, 2H), 4.65 (s, 2H).
   **¹³C NMR** (101 MHz, CDCl₃): δ/ppm = 25.7 (CH), 31.8 (C), 37.0 (3CH), 37.3 (3CH), 37.8 (3CH₂), 39.7 (3CH₂), 80.5 (CH₂).
   **IR** (neat): ṽ/cm⁻¹ = 3328, 3247, 2882, 2845, 1354, 1160, 974, 957, 846, 576, 552.
   **HRMS:** m/z = 297.13930 (calculated for C₁₅H₂₃NO₃S m/z = 297.13986)
physical data of substance **1g** (cf. Fig. 17A / Fig. 17B):
   **Yield:** 165 mg (65 %, 0.69 mmol scale)
   **R.f.** 0.32 (hexane/EtOAc 2/1, silica gel)
   m.p. 179.5 - 180.0 °C (crystallized from EtOAc)
   **¹H NMR** (600 MHz, CDCl₃): δ/ppm = 1.53 - 1.58 (m, 12H), 1.77 - 1.78 (m, 6H), 2.06 (s, 3H), 3.73 (s, 2H), 3.83 (s, 2H), 4.84 (s, 2H).
   **¹³C NMR** (151 MHz, CDCl₃): δ/ppm = 21.1 (C), 31.2 (C), 31.6 (C), 36.7 (3CH), 36.8 (3CH), 39.1 (3CH₂), 39.5 (3CH₂), 73.5 (CH₂), 80.2 (CH₂), 171.6 (C).
   **IR** (neat): ṽ/cm⁻¹ = 3343, 3257, 2888, 1748, 1747, 1361, 1247, 1226, 1160, 1036, 972, 953, 933,848,573,550.
   **HRMS:** m/z = 369.15938 (calculated for C₁₈H₂₇NO₅S m/z = 369.16099)
physical data of substance **1h** (cf. Fig. 18A / Fig. 18B):
   **Yield:** 100 mg (24 %, 1.4 mmol scale)
   **R.f.** 0.39 (hexane/EtOAc 2/1, silica gel)
   **m.p.** 179.1 - 179.6 °C (crystallized from EtOAc)
   **¹H NMR** (400 MHz, CDCl₃): δ/ppm = 1.48 - 2.01 (m, 19H), 4.19 (s, 2H), 4.69 (s, 2H).
   **¹³C NMR** (101 MHz, CDCl₃): δ/ppm = 25.7 (CH), 27.0 (CH), 32.9 (2CH₂), 37.1 (2CH), 37.2 (C), 37.7 (CH), 37.8 (2CH₂), 38.1 (2CH), 38.8 (CH₂), 40.3 (CH₂), 76.9 (CH₂).
   **IR** (neat): ṽ/cm⁻¹ = 3365, 3269, 2910, 1360, 1170, 967, 946, 927, 840, 562, 548, 527.
   **HRMS:** m/z = 297.13911 (calculated for C₁₅H₂₃NO₃S m/z = 297.13986)

### Synthesis of cyclic sulfamidates (2) by intramolecular amination reaction

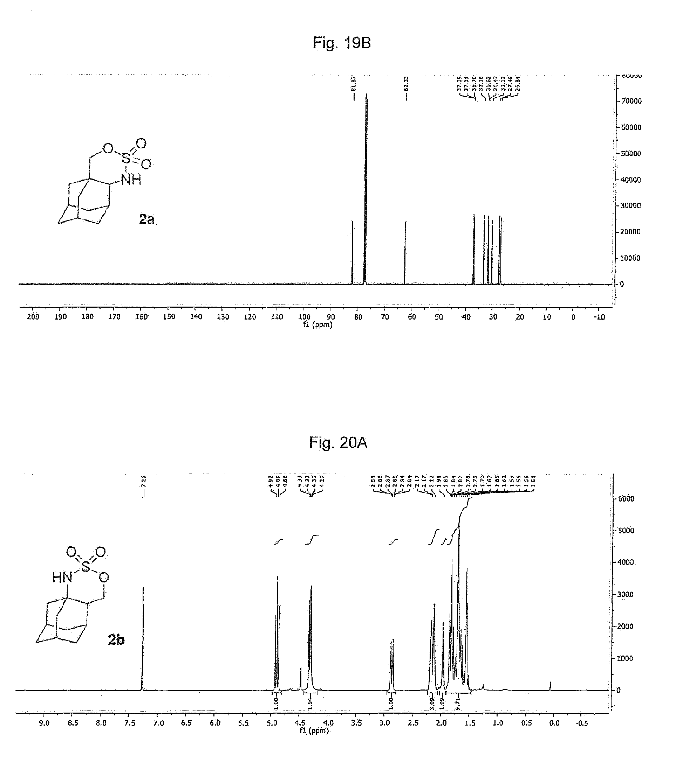

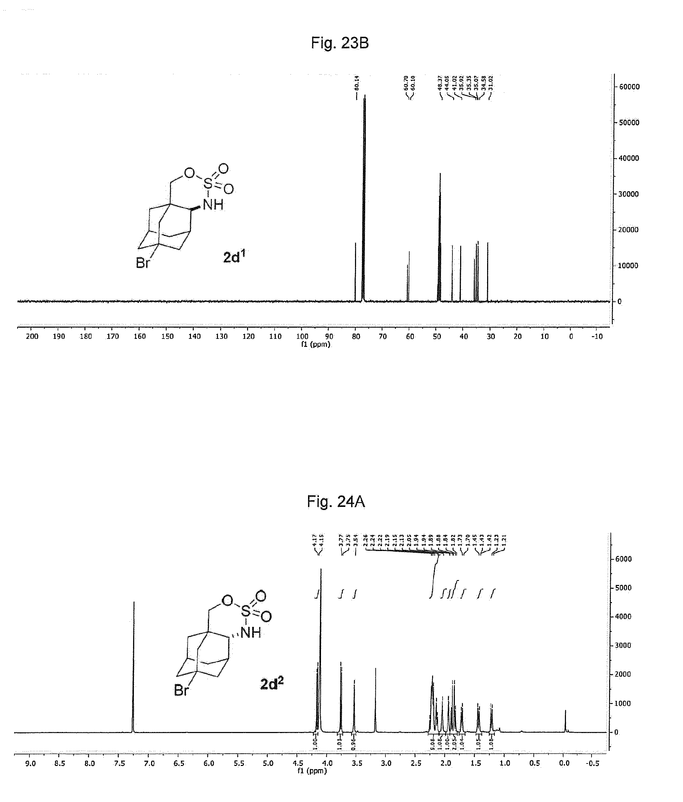

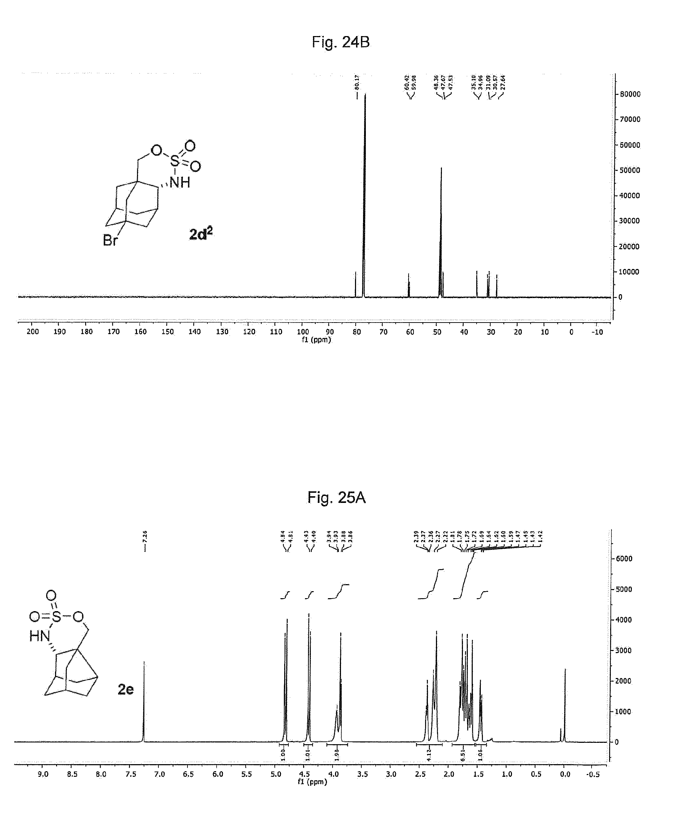

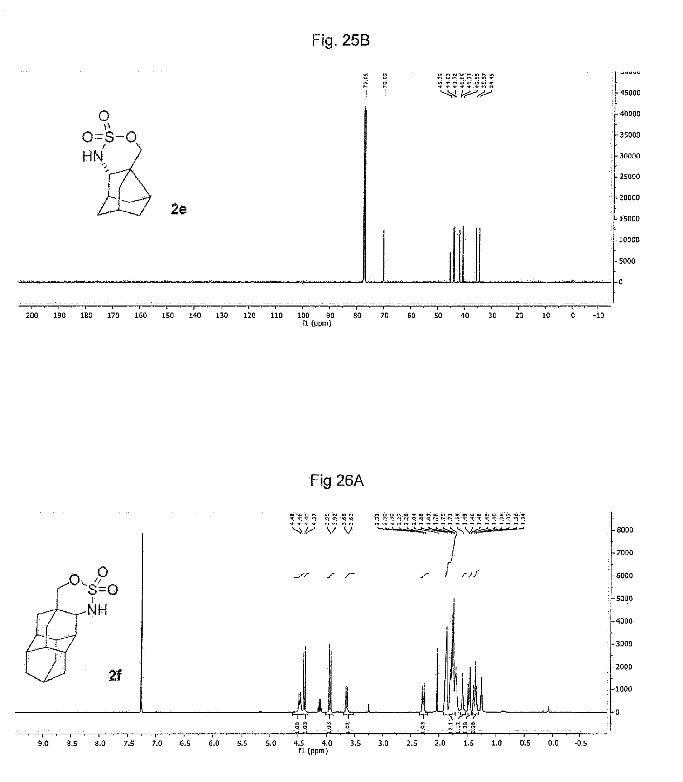

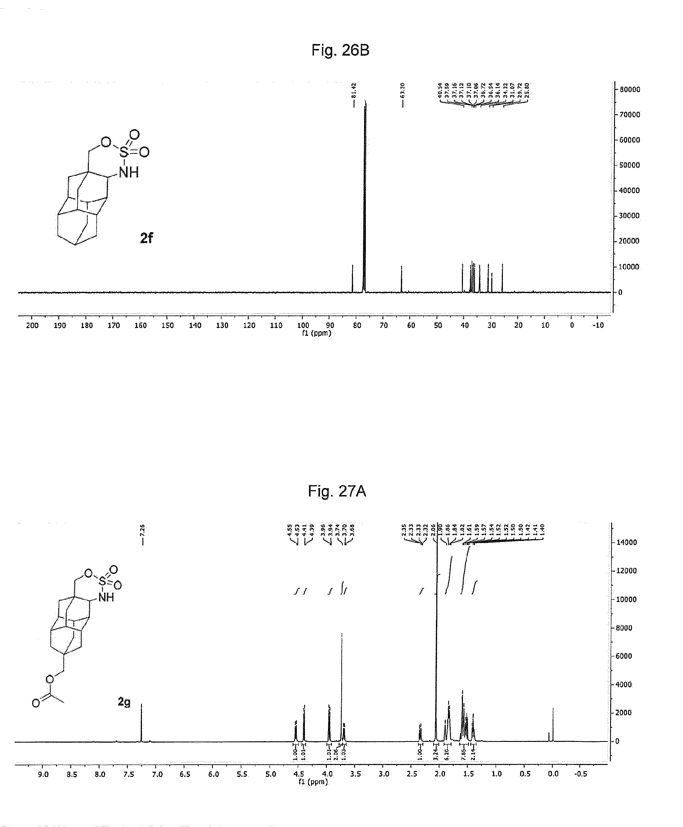

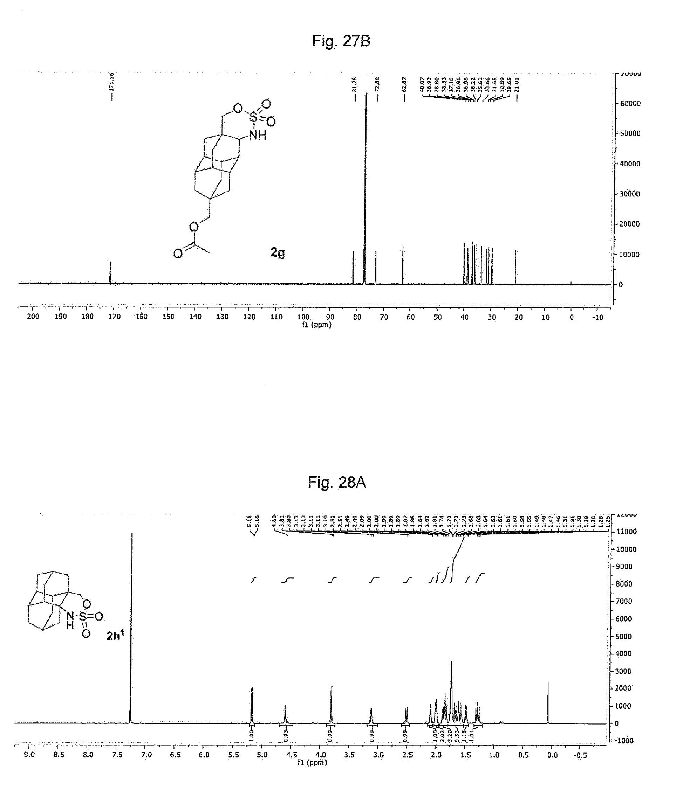

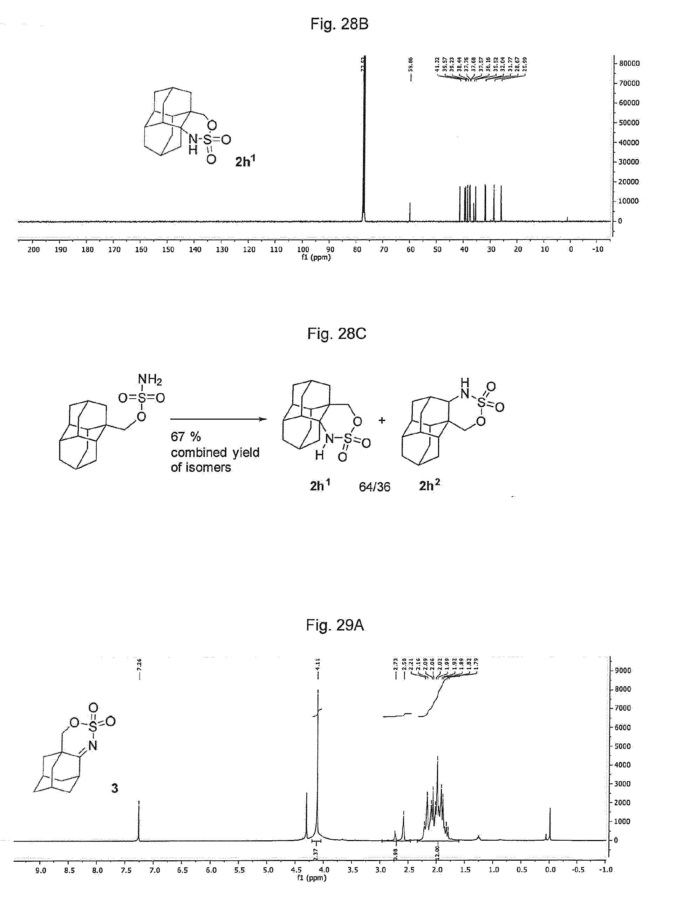

2.04 mmol of (1), iodobenzene 1,1-diacetate (880 mg, 2.73 mmol), Rh₂(OAc)₄ (9 mg, 0.024 mol), and MgO (200 mg, 5 mmol) were suspended in dry CH₂Cl₂ and the reaction mixture was heated to 40 °C under argon. The reaction was stirred for 3 hours at this temperature. After this time reaction was let to cool down to 25 °C and directly poured on silica gel. Silica gel was washed with hexane/ EtOAc 1/1 mixture to filter out the product from the rhodium catalyst and salts. The organic fraction was evaporated under vacuo and product was washed with hexane to remove iodobenzene. Colorless solid crystalline product was used for next step or purified by column chromatography on silica gel in mobile phase stated under each compound. All products (2) were crystallized from EtOAc and their structure was confirmed by diffraction analysis. In case of compound 2c the quality of data was not sufficient for publishing and the compound was oxidized to corresponding 2-keto derivative to reconfirm the structure.
physical data of substance **2a** (cf. Fig. 19A / Fig. 19B):
   **Yield:** 3.77 g (95 %, 16.3 mmol scale)
   **R.f.** 0.42 (hexane/EtOAc 2/1, silica gel)
   **m.p.** 170.7 - 171.4 °C (crystallized from EtOAc)
   **¹H NMR** (400 MHz, CDCl₃): δ/ppm = 1.31 - 1.43 (m, 2H), 1.45 - 1.56 (m, 1 H), 1.59 - 1.83 (m, 5H), 1.84 - 1.93 (m, 1 H), 1.94 - 2.14 (m, 3H), 2.3 (dd, *J* = 13.4, 2.4 Hz, 1H), 3.71 *(d, J* = 10.6 Hz, 1 H), 3.89 (d, *J =* 11.3 Hz, 1 H), 4.34 (d, *J =* 11.3 Hz, 1 H), 4.56 (d, *J =* 10.6 Hz, 1H).
   **¹³C NMR** (101 MHz, CDCl₃): δ/ppm = 26.8 (CH), 27.5 (CH), 30.1 (CH₂), 31.4 (C), 31.6 (CH), 33.2 (CH₂), 36.8 (CH₂), 37.0 (CH₂), 37.1 (CH₂), 62.3 (CH), 81.9 (CH₂).
   **IR** (neat): ṽ/cm⁻¹ = 3279, 2914, 2861, 1457, 1412, 1361, 1184, 1149, 1053, 1038, 979, 939, 905, 789, 707, 663, 637, 573, 566, 519, 504.
   **HRMS:** m/z = 243.09258 (calculated for C₁₁H₁₇NO₃S m/z = 243.09291)
physical data of substance **2b** (cf. Fig. 20A / Fig. 20B):
   **Yield:** 270 mg (90 %, 1.22 mmol scale)
   **R.f.** 0.40 (hexane/EtOAc 2/1, silica gel)
   m.p. 147.1 -148.0 °C (crystallized from EtOAc)
   **¹H NMR** (400 MHz, CDCl₃): δ/ppm = 1.46 - 1.90 (m, 9H), 1.92 - 1.97 (m, 1 H), 2.05 - 2.23 (m, 3H), 2.86 (dt, *J* = 13.2, 3.7 Hz, 1 H), 4.31 (dd, *J* = 11.5, 4.3 Hz, 2H), 4.81 - 4.97 (m, 1 H).
   **¹³C NMR** (101 MHz, CDCl₃): δ/ppm = 29.0 (CH), 29.4 (CH), 30.5 (CH), 30.9 (CH₂), 35.8 (CH₂), 36.4 (CH₂), 38.1 (CH₂), 44.7 (CH₂), 45.2 (CH), 56.5 (C), 73.4 (CH₂).
   **IR** (neat): ṽ/cm⁻¹ = 3248, 2918, 2855, 1454, 1431, 1366, 1337, 1190, 1153, 1126, 1094, 1076, 1055, 998, 961, 944, 916, 886, 837, 775, 728, 685, 660, 635, 579, 536.
   **HRMS:** m/z = 243.09321 (calculated for C₁₁H₁₇NO₃S m/z = 243.09291)
physical data of substance 2c (cf. Fig. 21A / Fig. 21 B):
   **Yield:** 198 mg (72 %, 1 mmol scale)
   **R.f.** 0.51 (hexane/EtOAc 2/1, silica gel)
   **m.p.** 178.8 -179.8 °C (crystallized from EtOAc)
   **¹H NMR** (400 MHz, CDCl₃): δ/ppm = 0.88 (s, 3H), 0.90 (s, 3H), 0.96 - 1.15 (m, 3H), 1.16-1.43 (m, 4H), 1.46 - 1.48 (m, 2H), 1.95 -2.06 (m, 2H), 3.58 (d, *J* = 10.5 Hz, 1 H), 3.87 (d, *J* = 11.2 Hz, 1 H), 4.38 (d, *J* = 11.2 Hz, 1 H), 4.45 (d, *J* = 10.4 Hz, 1 H).
   **¹³C NMR** (101 MHz, CDCl₃): δ/ppm = 29.6 (CH₃), 29.9 (CH₃), 30.6 (C), 31.3 (C), 32.8 (C), 32.9 (CH), 36.5 (CH₂), 39.5 (CH₂), 42.9 (CH₂), 43.1 (CH₂), 51.3 (CH₂), 61.3 (CH), 81.5 (CH₂).
   **IR** (neat): ṽ/cm⁻¹ = 3280, 2909, 2861, 1456, 1407, 1358, 1316, 1284, 1188, 1154, 1069, 1049, 1032, 959, 939, 909, 872, 783, 702, 612, 581, 550.
   **HRMS:** m/z = 271.12361 (calculated for C₁₃H₂₁NO₃S m/z =271.12421)
physical data of oxidation product of substance 2c (cf. Fig. 22A / Fig. 22B):
   Colorless non crystalline solid
   **R.f.** 0.7 (hexane/EtOAc 1/1, silica gel)
   **¹H NMR** (600 MHz, CDCl₃): δ/ppm = 0.95 (s, 6H), 1.35 (dd, *J* = 13.0, 4.1 Hz, 2H), 1.46 - 1.57 (m, 2H), 1.64 (d, *J* = 13.2 Hz, 2H), 1.70 - 1.80 (m, 4H), 2.35 (s, 1 H), 2.46 - 2.53 (m, 1H), 3.41 (s, 2H).
   **¹³C NMR** (151 MHz, CDCl₃): δ/ppm = 28.7 (2CH₃), 31.4 (2C), 45.0 (2CH₂), 46.7 (CH), 47.5 (2CH₂), 50.7 (CH₂), 50.8 (C), 67.9 (CH₂), 221.2 (C).
   **IR** (neat): ṽ/cm⁻¹ = 3455, 2947, 2923, 2865, 2849, 1709, 1457, 1377, 1357, 1114, 1053, 1038, 1006, 979, 911, 879, 556.
   **HRMS:** m/z = 208.14687 (calculated for C₁₃H₂₀O₂ m/z = 208.14633)
physical data of substance **2d:**
   **Yield:** 150 mg (92 %, 0.51 mmol scale, 1/1 mixture of two regioisomers)
physical data of substance **2d¹** (cf. Fig. 23A / Fig. 23B):
   Separated by HPLC (column: RP-18 (Lichrosorb 10 µm), 8 x 250 mm, mobile phase: 55% MeOH / 45% H₂0, flow: 4 mL/min)
   Retention time: 9 min
   Structure confirmed by diffraction analysis
   **R.f.** 0.23 (hexane/EtOAc 2/1, silica gel)
   **m.p.** 217.5 - 217.8 °C (crystallized from EtOAc)
   **¹H NMR** (400 MHz, CDCl₃/MeOD): δ/ppm = 1.27 - 1. 48 (m, 2H), 1.60 - 1.92 (m, 3H), 1.97 - 2.29 (m, 5H), 2.34 (d, *J* = 10.5 Hz, 1 H), 2.81 (dt, *J* = 12.8, 2.3 Hz, 1 H), 3.53 (s, 1 H), 3.82 (d, *J* = 11.5 Hz, 1 H), 4.25 (d, *J* = 11.5 Hz, 1 H).
   **¹³C NMR** (101 MHz, CDCl₃/MeOD): δ/ppm = 31.0 (CH), 34.6 (CH₂), 35.1 (CH₂), 35.4 (CH), 35.9 (C), 41.0 (CH₂), 44.1 (CH₂), 48.4(CH₂), 60.1 (CH), 60.7 (C), 80.1 (CH₂).
   IR (neat): ṽ/cm⁻¹ = 3274, 2910, 1487, 1455, 1409, 1362, 1314, 1271, 1188, 1152, 1135, 1062, 1045, 1018, 980, 946, 913, 896, 845, 822, 791, 763, 710, 686, 667, 650, 578, 568, 521,505.
   **HRMS:** m/z = 321.00225 (calculated for C₁₁H₁₆NBrO₃S m/z = 321.00343)
physical data of substance **2d²** (cf. Fig. 24A / Fig. 24B):
   Separated by HPLC (column: RP-18 (Lichrosorb 10 µm), 8 x 250 mm, mobile phase: 55% MeOH / 45% H₂0, flow: 4 mL/min)
   Retention time: 11 min
   **R.f.** 0.23 (hexane/EtOAc 2/1, silica gel)
   **m.p.** 198.0 -198.4 °C (crystallized from EtOAc)
   **¹H NMR** (600 MHz, CDCl₃/MeOD): δ/ppm = 1.21 - 1.23 (m, 1 H), 1.42 - 1.45 (m, 1 H), 1.72 (d, *J* = 13.8 Hz, 1 H), 1.79 - 1.92 (m, 2H), 1.92 - 1.98 (m, 1 H), 2.05 (s, 1 H), 2.10 - 2.28 (m, 5H), 3.54 (s, 1 H), 3.76 (d, *J* = 11.3 Hz, 1H), 4.16 (d, *J* = 11.3 Hz, 1 H).
   **¹³C NMR** (151 MHz, CDCl₃/MeOD): δ/ppm = 27.6 (CH₂), 30.6 (CH), 31.1 (CH2), 35.0 (C), 35.1 (CH), 47.5 (CH₂), 47.7 (CH₂), 48.4 (CH2), 60.0 (C), 60.4 (CH), 80.2 (CH₂).
   **IR** (neat): ṽ/cm⁻¹ = 3279, 2938, 1460, 1369, 1190, 1143, 1079, 1049, 999, 936, 913, 852, 829,781,688,598,529.
   **HRMS:** m/z = 321.00225 (calculated for C₁₁H₁₆NBrO₃S m/z = 321.00343)
physical data of substance **2e** (cf. Fig. 25A / Fig. 25B):
   **Yield:** 100 mg (85 %, 0.51 mmol scale)
   **R.f.** 0.33 (hexane/EtOAc 2/1, silica gel)
   **m.p.** 196.5 - 197.0 °C (crystallized from EtOAc)
   **¹H NMR** (400 MHz, CDCl₃): δ/ppm = 1.34 - 1.53 (m, 1 H), 1.55 - 1.94 (m, 6H), 2.10 - 2.55 (m, 4H), 3.74 - 4.10 (m, 2H), 4.42 (d, *J* = 11.8 Hz, 1 H), 4.82 (d, *J* = 11.8 Hz, 1 H).
   **¹³C NMR** (101 MHz, CDCl₃): δ/ppm = 34.5 (CH₂), 35.6 (CH), 40.6 (CH₂), 41.7 (CH), 41.8 (CH), 43.7 (CH₂), 44.0 (CH₂), 45.4 (C), 70.0 (CH), 77.1 (CH₂).
   **IR** (neat): ṽ/cm⁻¹ = 3312, 2933, 1470, 1416, 1358, 1336, 1318, 1185, 1076, 1024,958,921, 783, 708, 685, 593, 531.
   **HRMS:** m/z = 229.07694 (calculated for C₁₀H₁₅NO₃S m/z = 229.07726)
physical data of substance **2f** (cf. Fig. 26A / Fig. 26B):
   **Yield:** 56 mg (96 %, 0.2 mmol scale)
   **R.f.** 0.44 (hexane/EtOAc 2/1, silica gel)
   **m.p.** 213.5 - 214.0 °C (crystallized from EtOAc)
   **¹H NMR** (400 MHz, CDCl₃): δ/ppm = 1.31 - 1.47 (m, 3H), 1.50 -1.88 (m, 13H), 2.26 - 2.31 (m, 1 H), 3.64 (d, *J* = 10.7 Hz, 1 H), 3.94 (d, *J* = 11.2 Hz, 1 H), 4.39 (d, *J* = 11.2 Hz, 1 H), 4.47 (d, *J* = 10.6 Hz, 1 H).
   **¹³C NMR** (101 MHz, CDCl₃): δ/ppm = 25.8 (CH), 29.7 (C), 31.1 (CH), 34.2 (CH₂), 36.1 (CH), 36.5 (CH₂), 36.7 (CH), 37.0 (CH₂), 37.1 (CH), 37.1 (CH₂), 37.2 (CH), 37.6 (CH₂), 40.5 (CH), 63.2 (CH), 81.4 (CH₂).
   **IR** (neat): ṽ/cm⁻¹ = 3265, 2889, 1461, 1440, 1413, 1360, 1191, 1113, 1062, 1029, 972, 937, 905, 794, 726, 702, 634, 602, 565, 532.
   **HRMS:** m/z = 295.12391 (calculated for C₁₅H₂₁NO₃S m/z = 295.12421)
physical data of substance **2g** (cf. Fig. 27A / Fig. 27B):
   **Yield:** 90 mg (95 %, 0.25 mmol scale)
   **R.f.** 0.71 (CH₂Cl₂/EtOAc 2/1, silica gel)
   **m.p.** 178.5 -179.0 °C (crystallized from EtOAc)
   **¹H NMR** (400 MHz, CDCl₃): δ/ppm = 1.34 - 1.45 (m, 2H), 1.48 - 1.64 (m, 7H), 1.79 - 1.92 (m, 6H), 2.06 (s, 3H), 2.29 - 2.37 (m, 1 H), 3.69 (d, *J =* 10.6 Hz, 1 H), 3.74 (s, 2H), 3.95 (d, *J* = 11.3 Hz, 1 H), 4.40 (d, *J* = 11.3 Hz, 1 H), 4.54 (d, *J* = 10.6 Hz, 1 H).
   **¹³C NMR** (101 MHz, CDCl₃): δ/ppm = 21.0 (CH₃), 29.7 (C), 30.9 (CH), 31.7 (C), 33.7 (CH₂), 35.6 (CH), 36.2(CH), 36.9 (CH), 36.9 (CH), 37.1 (CH₂), 38.3 (CH₂), 38.8 (CH₂), 38.9 (CH₂), 40.1 (CH), 62.9 (CH), 72.9 (CH₂), 81.3 (CH₂), 171.4 (C).
   **IR** (neat): ṽ/cm⁻¹ = 3284, 2896, 2849, 1742, 1448, 1412, 1355, 1250, 1219, 1189, 1038, 972, 932, 912, 878, 793, 696, 676, 649, 632, 566, 534.
   **HRMS:** m/z = 367.14337 (calculated for C₁₈H₂₅NO₅S m/z = 367.14534)
physical data of substance **2h¹** (cf. Fig. 28A / Fig. 28B, Fig. 28C):
   **Yield:** 40 mg **of both isomers** (67 %, 0.16 mmol scale)
   Major isomer **2h¹** was separated using column chromatography (eluted as first, lower retention time on silica gel column) and fully characterized.
   **R.f.** 0.1 (hexane/EtOAc 10/1, silica gel)
   **m.p.** 199.2 - 200 °C (crystallized from EtOAc)
   **¹H NMR** (600 MHz, CDCl₃): δ/ppm = 1.19 - 1.34 (m, 2H), 1.48 (d, *J =* 13.5 Hz, 1 H), 1.52-1. 78 (m, 7H), 1.79 - 1.93 (m, 3H), 1.94 - 2.03 (m, 2H), 2.07 - 2.10 (m, 1 H), 2.50 (d, *J =* 13.7 Hz, 1 H), 3.12 (d, *J =* 13.7 Hz, 1 H), 3.80 (d, *J* = 11.4 Hz, 1 H), 4.60 (s, 1 H), 5.17 (d, *J =* 11.4 Hz, 1H).
   **¹³C NMR** (151 MHz, CDCl₃): δ/ppm = 26.0 (CH), 28.7 (CH), 31.8 (CH₂), 32.0 (CH₂), 35.5 (CH₂), 36.2 (CH), 37.6 (CH₂), 37.7(CH₂), 37.8 (CH₂), 38.4 (CH), 39.2 (CH), 39.6 (CH), 41.3 (CH), 59.9 (C), 77.5 (CH₂).
   **IR** (neat): ṽ/cm⁻¹ = 3294, 2921, 2875, 2854, 1477, 1447, 1395, 1331, 1242, 1187, 1123, 1029, 987, 947, 914, 795, 775, 747, 700, 668, 614, 598, 559.
   **HRMS:** m/z = 295.12456 (calculated for C₁₅H₂₁NO₃S m/z = 295.12421)

### Oxidation of sulfamidate moiety to sulfimidate (3)

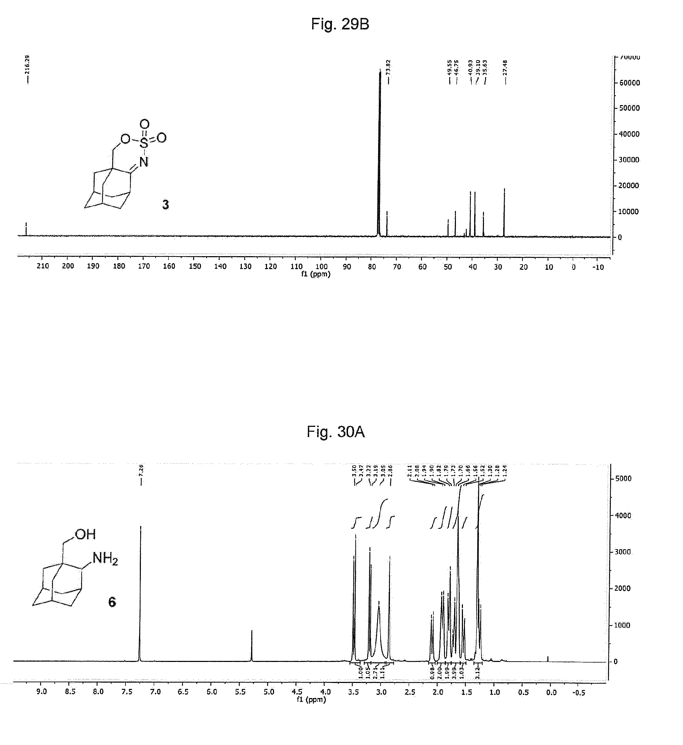

2a (480 mg, 1.98 mmol) was added to the solution of KOH (120 mg, 2.14 mmol) and KMnO₄ (352 mg, 2.23 mmol) in water (6 mL). The reaction mixture was heated to 70 °C and vigorously stirred for 2 hours. Then the reaction mixture was cooled to 25 °C, neutralised using 0.5M HCl and product was extracted using EtOAc. Organic fraction was dried using MgSO₄, filtered and solvent was evaporated under vacuo. Product 3 was used directly for further steps.
physical data of substance 3 (cf. Fig. 29A / Fig. 29B):
   **Yield:** 470 mg (98 %, 1.98 mmol scale)
   Non crystalline solid
   **R.f.** 0.55 (Et₂O, silica gel)
   **¹H NMR** (400 MHz, CDCl₃): δ/ppm = 1.79 - 2.21 (m, 12H), 2.58 - 2.68 (m, 1H), 4.11 (s, 2H).
   **¹³C NMR** (101 MHz, CDCl₃): δ/ppm = 27.5 (2CH), 35.6 (CH₂), 39.1 (2CH₂), 40.9 (2CH₂), 46.8 (CH), 49.6 (C), 73.8 (CH₂), 216.3 (C).
   **IR** (neat): ν̃/cm⁻¹ = 2909, 2860, 1687, 1566, 1461, 1375, 1184, 991, 952, 844, 797, 784, 696,565,546.
   **HRMS:** m/z = 241.07689 (calculated for C₁₁H₁₅NO₃S m/z = 241.07726)

### Reductive cleavage of sulfamidate moiety, synthesis of amino alcohol (6)

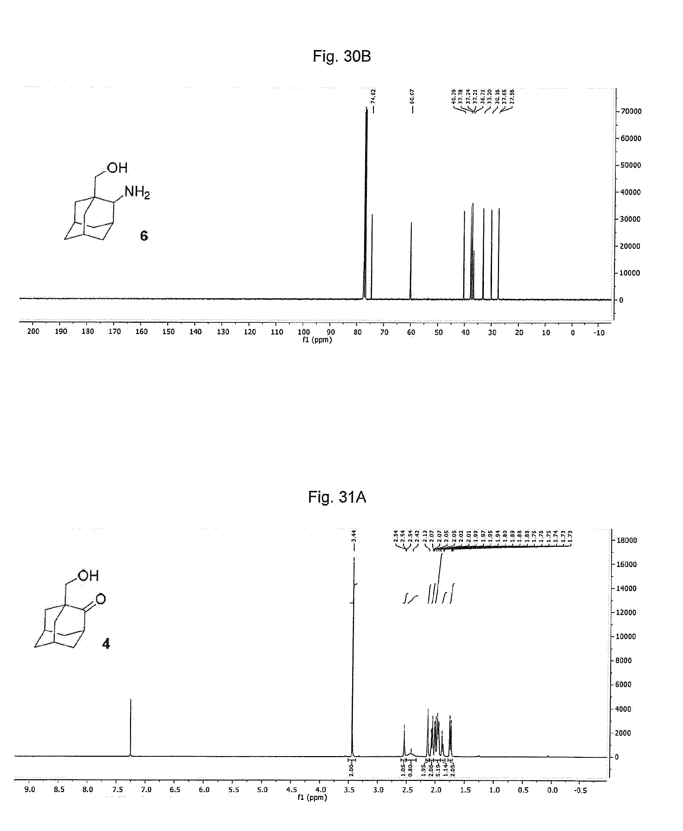

**2a** (486 mg, 2 mmol) was dissolved in dry toluene (20 mL) and 0.5M solution of Me₂N-AlH₃ in toluene (15 mL) was added dropwise at 0 °C under argon. Then the reaction mixture was heated to 120 °C and stirred at this temperature for 3 hours. After the reaction was cooled to 0 °C, quenched with water and 14 % H₂SO₄ was added (20 mL) and the mixture was treated for 1 h at 40 °C. Then the water layer was separated and neutralised using K₂CO₃. Product was extracted in EtOAc, solution was dried using MgSO₄, solvent evaporated under vacuo and amino alcohol **(6)** was purified using column chromatography on neutral alumina using CH₂Cl₂/MeOH 5/1 as mobile phase.
physical data of substance **6** (cf. Fig. 30A / Fig. 30B):
   **Yield:** 261 mg (72 %, 2 mmol scale) of colorless solid
   R.f. 0.05 (CH₂Cl₂/MeOH 5/1, silica gel)
   Non crystalline solid
   **¹H NMR** (400 MHz, CDCl₃): δ/ppm = 1.21 - 1.35 (m, 3H), 1.52 - 1.56 (m, 1 H), 1.66 - 1.73 (m, 4H), 1.79 - 1.82 (m, 2H), 1.90 - 1.94 (m, 2H), 2.10 (d, *J* = 13.2 Hz, 1H), 2.86 (s, 1H), 3.05 (bs, 3H), 3.20 (d, *J =* 10.9 Hz, 1H), 3.48 (d, *J =* 10.9 Hz, 1 H).
   **¹³C NMR** (101 MHz, CDCl₃): δ/ppm = 27.5 (CH), 27.6 (CH), 30.2 (CH₂), 33.2 (CH₂), 36.7 (C), 37.2 (CH₂), 37.2 (CH), 37.8 (CH₂), 40.4 (CH₂), 60.1 (CH), 74.6 (CH₂).
   **IR** (neat): ν̃/cm⁻¹ = 3287, 2906, 2852, 1578, 1452, 1385, 1216, 1150, 1036, 929, 603.
   **HRMS:** m/z = 181.14540 (calculated for C₁₁H₁₉NO m/z = 181.14666)

### Hydrolysis of sulfimidate (3) to keto alcohol (4)

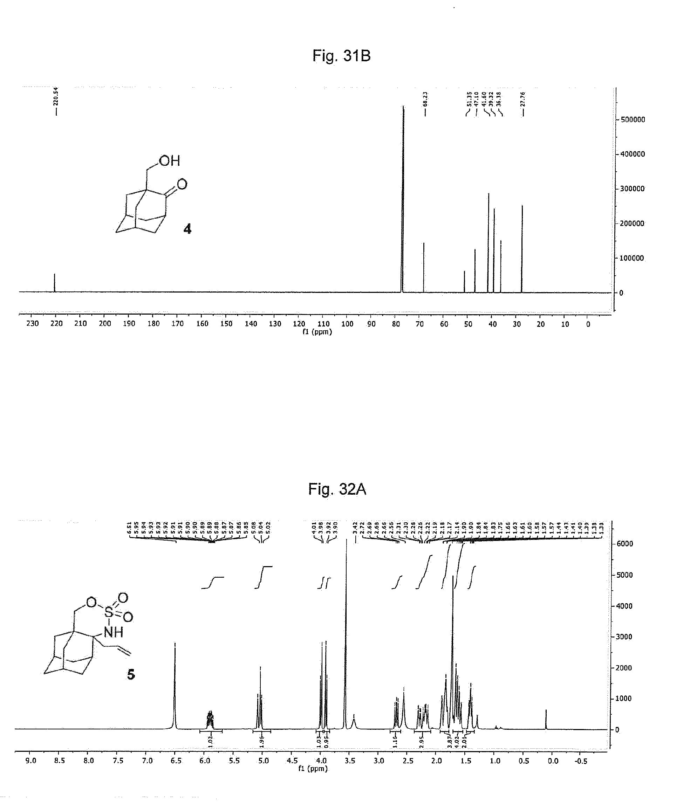

**3** (60 mg, 0.25 mmol) was suspended in water (3 mL) in microwave vial and 0.1 mL 48% HBF₄/H₂O was added. The reaction vessel was closed and the reaction mixture was heated to 120 °C and irradiated in microwave for 1 hour. Then the product was extracted in EtOAc and purified using column chromatography on silica gel with EtOAc as mobile phase to obtain 40 mg (91 %) of colorless non crystalline solid **4.**
physical data of substance **4** (cf. Fig. 31A / Fig. 31 B):
   **Yield:** 40 mg (91 %, 0.25 mmol scale)
   **R.f.** 0.45 (EtOAc, silica gel)
   Non crystalline solid
   **¹H NMR (600** MHz, CDCl₃): δ/ppm = 1.73 -1.75 (m, 2H), 1.83 -1.91 (m, 1 H), 1.91 - 2.03 (m, 5H), 2.06 (dd, J = 13.1, 3.0 Hz, 2H), 2.01 - 2.15 (m, 2H), 2.42 (bs, 1 H), 2.50 - 2.59 (m, 1 H), 3.44 (s, 2H).
   **¹³C NMR** (151 MHz, CDCl₃): δ/ppm = 27.8 (2CH), 36.4 (CH₂), 39.3 (2CH₂), 41.6 (2CH₂), 47.1 (CH), 51.4 (C), 68.2 (CH₂), 220.5 (C).
   **IR** (neat): ν̃/cm⁻¹ = 3448, 2914, 2854, 1705, 1455, 1399, 1359, 1317, 1296, 1217, 1156, 1124, 1044, 980, 962, 879, 622.
   **HRMS:** m/z = 180.11477 (calculated for C₁₁H₁₆O₂ m/z = 180.11503)

### Addition of allylmagnesium bromide on sulfimidate (3), synthesis of (5)

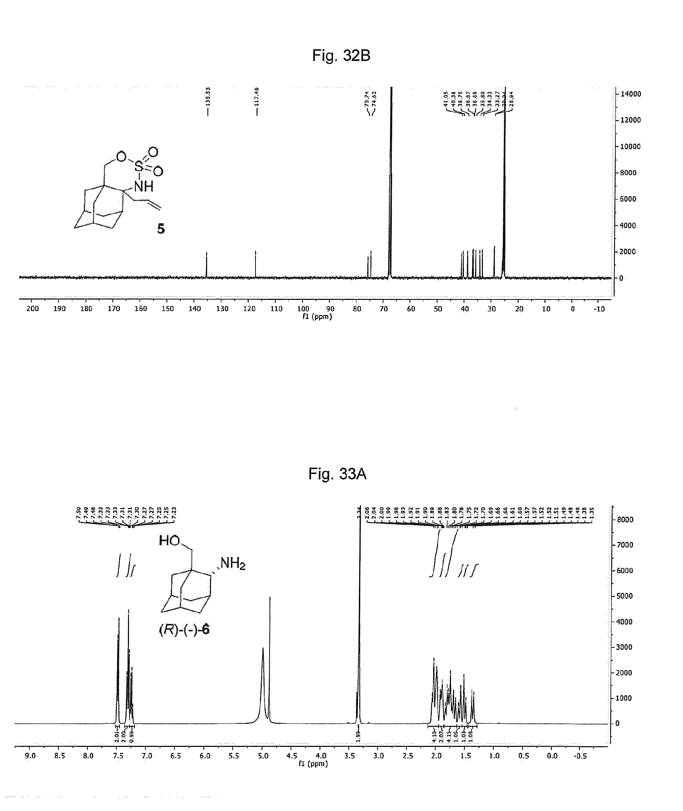

3 (100 mg, 0.42 mmol) was dissolved in dry Et₂O (4 mL) and the reaction mixture was cooled to 0 °C. 1 M solution of allyl magnesium bromide in Et₂O (0.63 mL) was added dropwise and the reaction mixture was stirred at 0 °C for 3 hours. Then the reaction was quenched using 0.5 M HCl (5 mL) and the product was extracted in EtOAc. Organic solution was dried using MgSO₄, solvent evaporated under vacuo and product **5** was purified using column chromatography on silica gel using hexane/EtOAc 1/1 as mobile phase to obtain 75 mg (63 %) of colorless crystalline solid **5.**
data of substance **5** (cf. Fig. 32A / Fig. 32B):
   **Yield:** 75 mg (63 %, 0.42 mmol scale)
   **R.f.** 0.27 (hexane/EtOAc 1/1, silica gel)
   **m.p.** 138.9 -139.5 °C (crystallized from EtOAc)
   **¹H NMR** (400 MHz, *d*-THF): δ/ppm = 1.34 - 1.47 (m, 2H), 1.57 - 1.93(m, 8H), 2.09 - 2.37 (m, 3H), 2.69 (dd, *J* = 14.2, 9.1 Hz, 1 H), 3.91 (d, *J* = 9.4 Hz, 1 H), 4.00 (d, *J* = 9.4 Hz, 1 H), 5.02 - 5.08 (m, 2H), 5.85 - 5.95 (m, 1 H).
   **¹³C NMR** (101 MHz, d-THF): δ/ppm = 28.9 (CH), 29.0 (CH), 33.3 (CH₂), 34.3 (CH₂), 35.8 (CH₂), 36.7 (CH), 36.9 (CH₂), 38.7 (CH₂), 40.4 (CH₂), 41.1 (C), 74.6 (CH₂), 75.7 (C), 117.5 (CH₂), 135.5 (CH).
   **IR** (neat): ν̃/cm⁻¹ = 3574, 3372, 3189, 3083, 2890, 2852, 1646, 1559, 1467, 1454, 1377, 1249, 1179, 1021, 1007, 983, 939, 857, 833, 796, 733, 669, 619, 553, 522.
   **HRMS:** m/z = 283.12372 (calculated for C₁₄H₂₁NO₃S m/z = 283.12421)

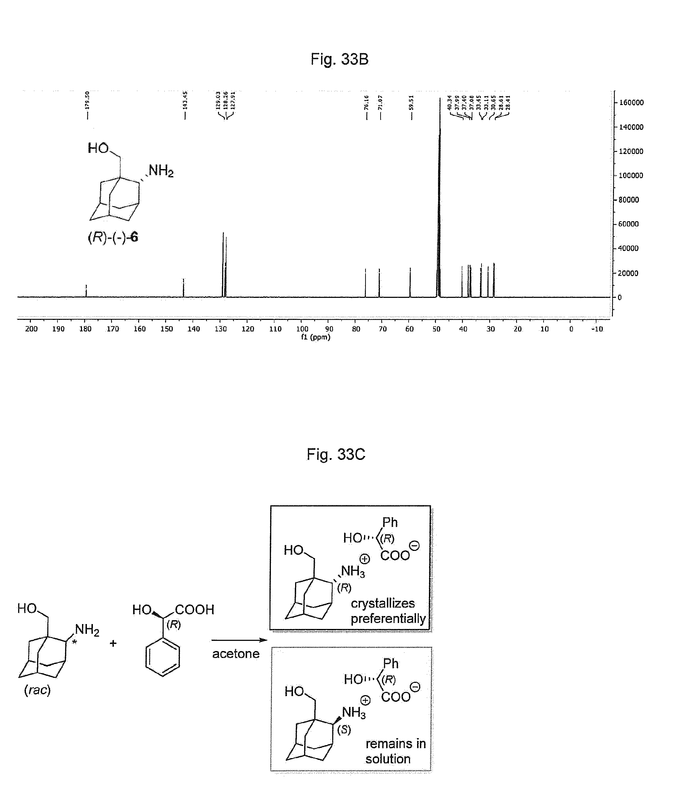

### Enantiomeric resolution of rac-(6) and absolute configuration determination (cf. Fig. 33C)

*Rac***-(6)** (80 mg, 0.44 mmol) was dissolved in acetone 3 mL and (*R*)-mandelic acid (67 mg, 0.44 mmol) was added. The mixture was heated till all material dissolved.

Then the flask was left to cool to 25 °C and closed was left overnight for crystallization.

Diastereomeric salt of (*R*)-6 with (*R*)-mandelic acid crystallized out of the solution. (*S*)-6 salt with (*R*)-mandelic acid remained dissolved. Crystals were separated (filtered) from the solution. The filtrate was concentrated. Absolute configuration of the crystalline salt (*R*)-6 with (R)-mandelic acid was determined using diffraction analysis. Separated salts were then neutralised using K₂CO₃ water solution and corresponding enantiomers of **6** were extracted in EtOAc. After drying with MgSO₄ and solvent evaporation, both of the enantiomers were separately subjected for crystallisation with 1 equivalent of corresponding enantiomers of mandelic acid. (*R*)-**6** (50 mg) with (*R*)-mandelic acid (42 mg). (*S*)**-6** (55 mg) with (*S*)-mandelic acid (46 mg). Second crystallisation and basic treatment provided enantiopure **6** for further syntheses. Optical rotation was measured.
data of substance **6** (cf. Fig. 33A / Fig. 33B):
   **Specific optical rotation:**
      (R)-**6**: [α]_{D}=-198 (c = 0.33; CHCl₃)
      (S)-**6**: [α]_{D}=+198 (c = 0.33; CHCl₃)
      Optical purity (higher 99%) was determined using HPLC with chiral stationary phase for derivatives (R)-8 and (S)-8.
      **¹H NMR** (400 MHz, MeOD): δ/ppm = 1.36 *(d, J =* 13.3 Hz, 1 H), 1.45 - 1.63 (m, 2H), 1.64-1.86 (m, 4H), 1.86 - 1.95 (m, 2H), 1.95 - 2.13 (m, 4H), 3.34 (s, 2H), 7.20 - 7.28 (m, 1H), 7.28 - 7.36 (m, 2H), 7.45 - 7.53 (m, 2H).
      **¹³C NMR** (101 MHz, MeOD): δ/ppm = 28.4 (CH), 28.6 (CH), 30.6 (CH₂), 33.1 (CH), 33.5 (CH₂), 37.1 (C), 37.4 (CH₂), 38.0 (CH₂), 40.3 (CH₂), 59.5 (CH), 71.1 (CH₂), 76.2 (CH), 127.9 (2CH), 128.3 (CH), 129.0 (2CH), 143.5 (CH), 179.5 (C).

### Cbz protection, preparation of (7) and its oxidation to beta amino acid (8)

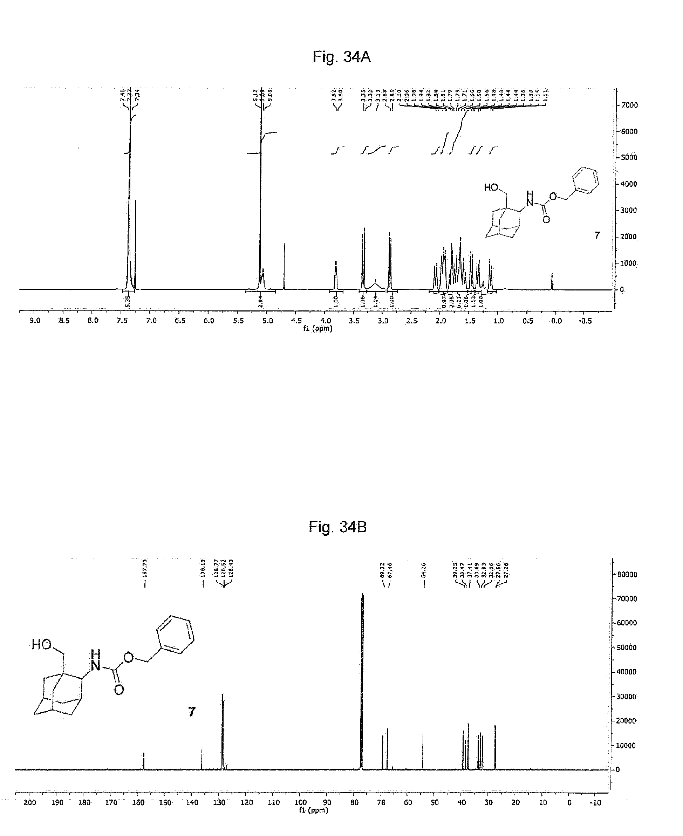

**6** (181 mg, 1 mmol) and Et₃N (278 µL, 2 mmol) were dissolved in 10 mL CH₂Cl₂. Solution was cooled to 0 °C and CbzCl (340 mg, 283 µL, 2 mmol) was added dropwise. Reaction mixture was stirred for 3 hours at 0 °C. Then was quenched with water and product was extracted using EtOAc. Organic fraction was dried over MgSO₄, solvent evaporated under vacuo (30 °C bath) and 7 was purified using column chromatography on silica gel using hexane/EtOAc 2/1 as mobile phase to obtain 205 mg (65%) of colorless non crystalline solid.
data of substance 7 (cf. Fig. 34A / Fig. 34B):
   **Yield:** 205 mg (65 %, 1 mmol scale)
   **R.f.** 0.25 (hexane/EtOAc 2/1, silica gel)
   Non crystalline solid
   **¹H NMR** (400 MHz, CDCl₃): δ/ppm = 1.13 *(d, J =* 12.9 Hz, 1H), 1.35 (d, *J* = 12.8 Hz, 1H), 1.41 - 1.52 (m, 1 H), 1.53 - 1.87 (m, 6H), 1.87 - 2.02 (m, 3H), 2.08 (d, *J* = 15.1 Hz, 1 H), 2.87 (d, *J* = 12.0 Hz, 1H), 3.13 (bs, 1H), 3.33 *(d, J =* 12.0 Hz, 1H), 3.81 (d, *J* = 8.3 Hz, 1H), 5.06 - 5.08 (m, 1 H), 5.12 (s, 2H), 7.34 - 7.40 (m, 5H).
   **¹³C NMR** (101 MHz, CDCl₃): δ/ppm = 27.3 (CH), 27.6 (CH), 32.1 (CH₂), 32.9 (CH), 33.7 (CH₂), 37.4 (CH₂), 38.5 (C), 39.3 (CH₂), 54.3 (CH), 67.5 (CH₂), 69.2 (CH2), 128.4 (2CH), 128.5 (CH), 128.8 (2CH), 136.2 (C), 157.7 (C).
   **IR** (neat): ν̃/cm⁻¹ = 3349, 3274, 2909, 2852, 1675, 1549, 1458, 1343, 1265, 1244, 1177, 1155, 1133, 1097, 1074, 1040, 1031, 937, 728, 634, 573.
   **HRMS:** m/z = 315.18226 (calculated for C₁₉H₂₅NO₃ m/z = 315.18344)

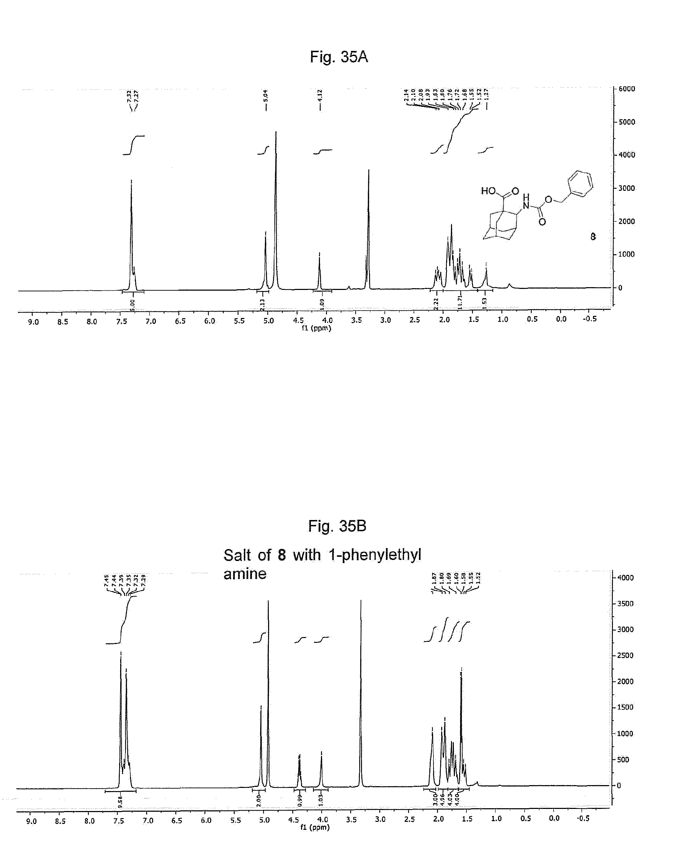

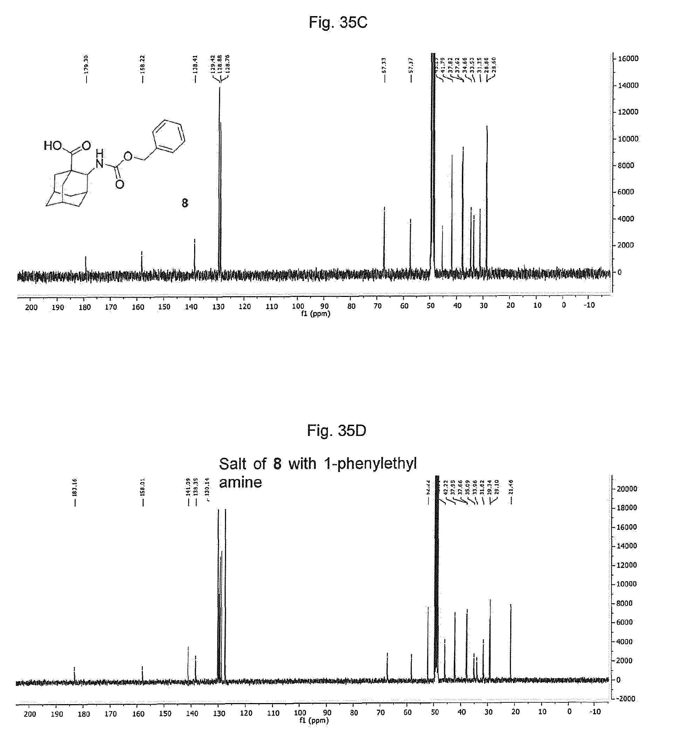

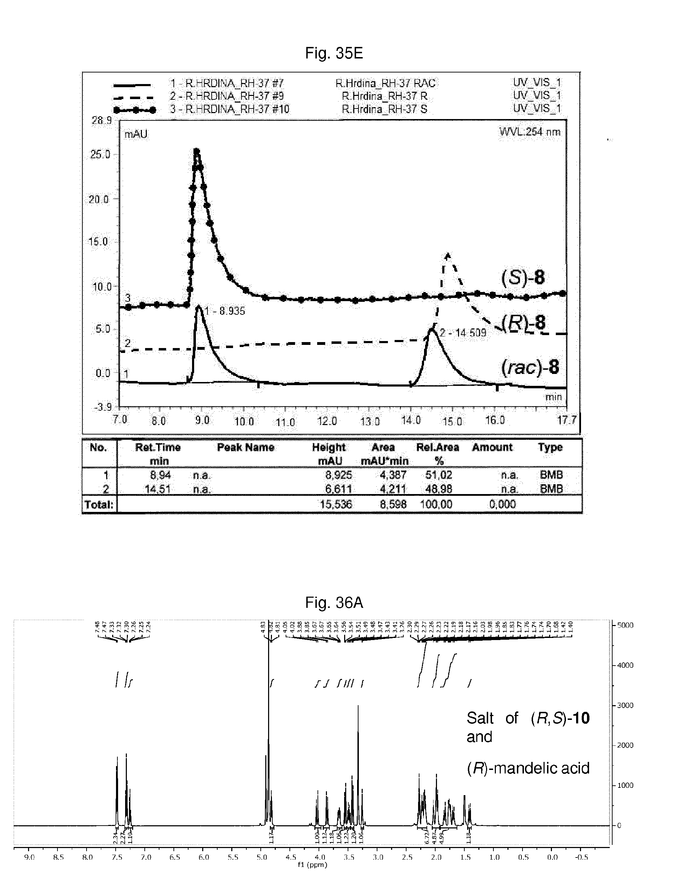

Compound 7 (100 mg, 0.32 mmol) was dissolved in 2 mL of acetone the solution was cooled to 0 °C. Jones reagent (CrO₃ 113 mg, H₂SO₄ 120 mg, 0.4 mL H₂O) was added dropwise during 10 minutes. The reaction mixture was stirred for 1 h at 0 °C. Then 15 g of ice was added and the product was extracted using 30 mL EtOAc. Organic solution was washed with 15 mL of water and then dried over MgSO₄, solvent was then evaporated under vacuo (30 °C bath) and compound 8 was purified by column chromatography on silica gel using hexane/EtOAc 1/1 as mobile phase.
data of substance **8** (cf. Fig. 35A / Fig. 35B / Fig. 35C / Fig. 35D):
   **Yield:** 55 mg (52 %, 0.32 mmol scale)
   **R.f.** 0.36 (hexane/EtOAc 1/1, silica gel)
   Non crystalline solid (compound may be stored as salt with 1-phenylethyl amine, crystalline solid)
   **¹H NMR** (400 MHz, *d*-MeOD): δ/ppm = 1.22 -1.28 (m, 1 H), 1.52 - 2.08 (m, 10H), 2.10 - 2.14 (m, 2H), 4.08 - 4.12 (m, 1 H), 5.02 - 5.17 (m, 2H), 7.25 - 7.36 (m, 5H).
Salt of **8** with 1-phenylethyl amine:
   **¹H NMR** (400 MHz, MeOD): δ/ppm = 1.52 -1.55 (m, 1 H), 1.59 (d, *J* = 6.8 Hz, 3H), 1.60 - 1.80 (m, 4H), 1.87 -1.93 (m, 5H), 2.00 - 2.10 (m, 3H), 4.01 (s, 1 H), 4.38 (q, *J* = 6.8 Hz, 1 H), 5.04 (s, 2H), 7.29 - 7.45 (m, 10 H).
   **¹³C NMR** (101 MHz, *d*-MeOD): δ/ppm = 28.6 (CH), 28.9 (CH), 31.3 (CH₂), 33.5 (CH₂), 34.7 (C), 37.6 (CH), 37.8 (CH₂), 41.8 (CH₂), 45.3 (CH₂), 57.4 (CH), 67.3 (CH₂), 128.8 (CH), 128.9 (2CH), 129.4 (2CH), 138.4 (C), 158.2 (C), 179.3 (C).
Salt of 8 with 1-phenylethyl amine:
   **¹³C NMR** (101 MHz, MeOD): δ/ppm = 21.5 (CH₃), 29.1 (CH), 29.4 (CH), 31.6 (CH₂), 34.0 (CH), 35.1 (CH₂), 37.7 (CH₂), 38.0 (CH₂), 42.2 (CH₂), 46.0 (C), 52.3 (CH), 58.3 (CH), 67.3 (CH₂), 127.6 (2CH), 128.9 (2CH), 129.4 (2CH), 129.7 (CH), 129.7(CH), 130.2 (2CH), 138.4 (C), 141.1 (C), 158.0 (C), 183.2 (C).
**IR** (neat): ν̃/cm⁻¹ = 3322, 2915, 2856, 2627, 1713, 1515, 1455, 1346, 1248, 1139, 1092, 1027, 994, 910, 832, 774, 733, 697, 664, 647, 605, 574.
   **HRMS:** m/z = 329.16165 (calculated for C₁₉H₂₃NO₄ m/z = 329.16271)
data of **(R)-(-)-8** (cf. Fig. 35E):
   **Specific optical rotation:** [α]_{D}= -6 (c = 1; CHCl₃)
HPLC with chiral stationary phase:
   Column: Chiralpak IA (250 mm x 4.6 mm)
   Mobile phase: 65% *n*-hexane/ 35% EtOAc
   Flow: 1 ml/min (pressure ∼ 30 atm)
   Detector: UV/VIS (254 nm)
   HPLC retention times:
   (*S*)-(+)-**8** : 8.94 min
   (*R*)-(-)-**8** : 14.5 min

### Synthesis of Vildagliptin analogs (10)

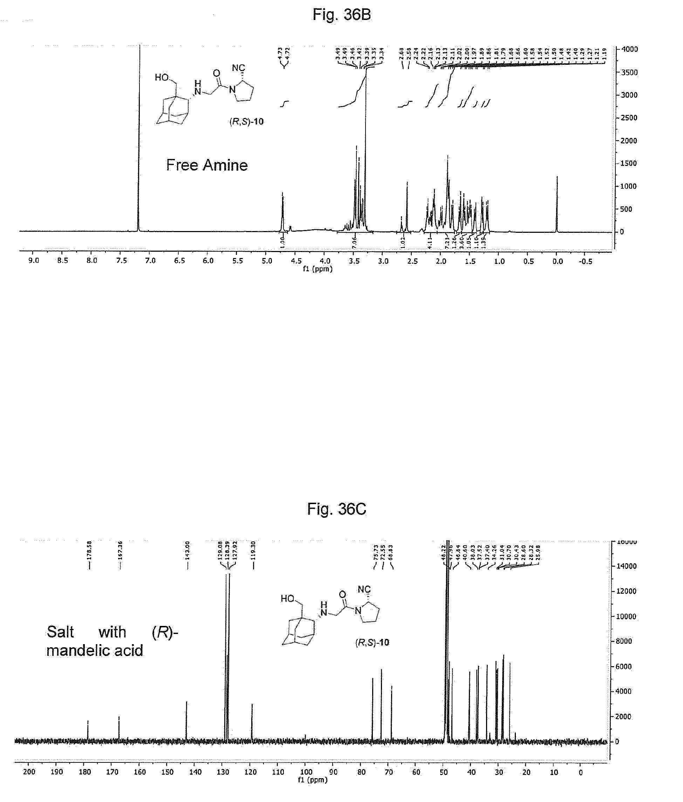

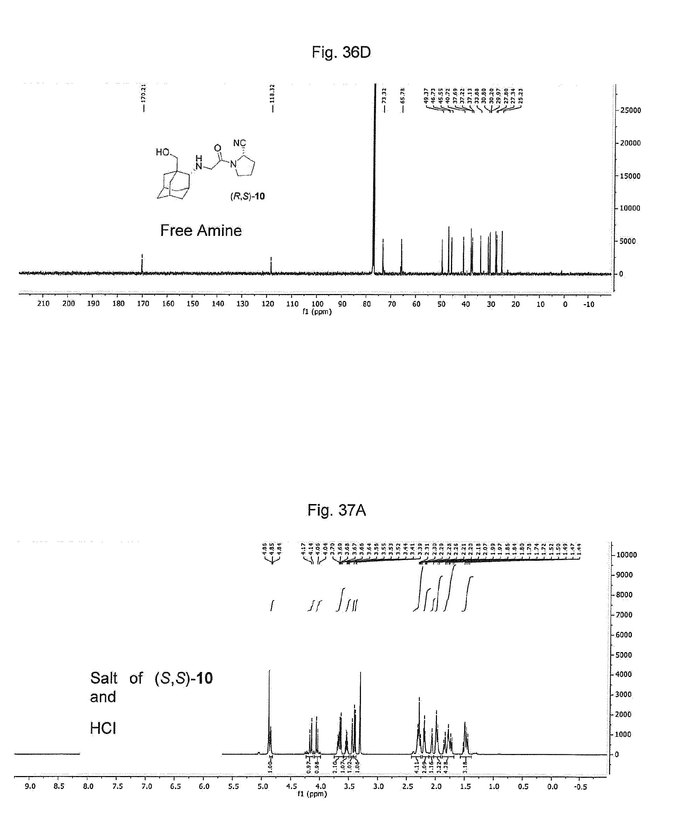

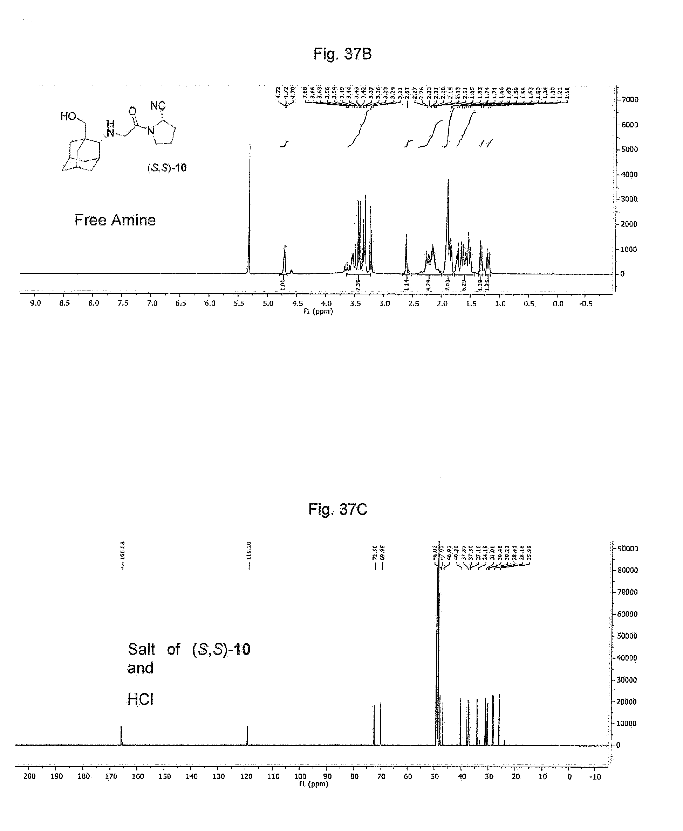

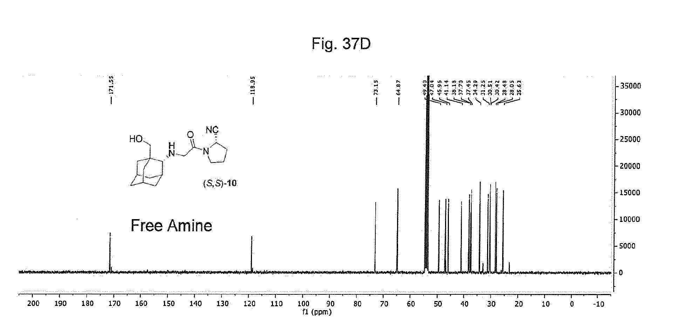

(*R*)-**6** or (*S*)-**6** (100 mg, 0.55 mmol) was dissolved in dry THF (2 mL), K₂CO₃ (113 mg) was added and solution of pyrrolidine 9 (47 mg, 0.27 mmol in 0.5 mL THF) was added dropwise. The reaction mixture was stirred for three days at 25 °C. Reaction was quenched with water (5 mL) and product was extracted in EtOAc. After drying the solution over MgSO₄, the solvent was evaporated under vacuo and the product was purified using column chromatography on silica gel using CH₂Cl₂/MeOH 10/1 as mobile phase. The corresponding compounds are characterized as free amine and as salts with acids. (*R,S*)-**10** was crystallized with (R)-mandelic acid and its structure was reconfirmed using diffraction analysis. (*S,S*)-**10** is characterized as free amine and as salt with HCl.
data of substance (R,S)-**10** (cf. Fig. 36A / Fig. 36B / Fig. 36C / Fig. 36D):
   **Yield:** 70 mg (82 %, 0.27 mmol scale)
   **R.f.** 0.10 (CH₂Cl₂/MeOH 10/1, silica gel)
   Free amine: non crystalline solid
   Salt of (*R,S*)-**10** and (R)-mandelic acid:
      **¹H NMR** (600 MHz, MeOD): δ/ppm = 1.41 (d, *J* = 13.4 Hz, 1 H), 1.63 - 1.91 (m, 5H), 1.93 - 2.05 (m, 5H), 2.15 - 2.31 (m, 6H), 3.26 (s, 1H), 3.42 (d, *J* = 10.9 Hz, 1H), 3.47 - 3.55 (m, 1 H), 3.55 *(d, J =* 10.9 Hz, 1 H), 3.66 (dd, *J* = 15.4 Hz, 5.9 Hz, 1 H), 3.86 (d, *J =* 16.4 Hz, 1 H), 4.03 (d, *J* = 16.4 Hz, 1 H), 4.82 (t, *J* = 5.6 Hz, 1H), 7.24 -7.26 (m, 1 H), 7.30 - 7.33 (m, 1H), 7.48 (d, *J* = 7.4 Hz, 2H).
Free amine:
   **¹H NMR** (600 MHz, CDCl₃): δ/ppm = 1.20 (d, *J* = 12.8 Hz, 1 H), 1.27 - 1.29 (m, 1H), 1.45 - 1.64 (m, 3H), 1.66 - 1.68 (m, 1 H), 1.76 - 2.06 (m, 6H), 2.06 - 2.28 (m, 4H), 2.58 - 2.68 (m, 1 H), 3.25 - 3.55 (m, 7H), 4,72 - 4.73 (m, 1 H).
Salt with (*R*)-mandelic acid:
   **¹³C NMR** (151 MHz, MeOD): δ/ppm = 26.0 (CH₂), 28.3 (CH), 28.6 (CH), 30.4 (CH), 30.7 (CH₂), 31.0 (CH₂), 34.3 (CH₂), 37.4 (CH₂), 37.5 (C), 38.0 (CH₂), 40.6 (CH₂), 46.8 (CH₂), 47.9 (CH), 48.2 (CH₂), 68.8 (CH), 72.5 (CH₂), 75.7 (CH), 119.3 (C), 127.9 (2CH), 128.4 (CH), 129.1 (2CH), 143.0 (C), 167.4 (C), 178.6 (C).
Free amine:
   **¹³C NMR** (151 MHz, CDCl₃): δ/ppm = 25.2 (CH₂), 27.3 (CH), 27.8 (CH), 30.0 (CH₂), 30.2 (CH), 30.8 (CH₂), 33.9 (CH₂), 37.1 (CH₂), 37.2 (C), 37.7 (CH₂), 40.7 (CH₂), 45.6 (CH₂), 46.7 (CH), 49.4 (CH₂), 65.8 (CH), 73.3 (CH₂), 118.3 (C), 170.2 (C).
**IR** (neat): ν̃/cm⁻¹ = 3383, 2905, 2850, 1658, 1414, 1320, 1264, 1152, 1041, 998, 909, 735.
**HRMS:** m/z = 317.20940 (calculated for C₁₈H₂₇N₃O₂ m/z = 317.21033)
data of substance (S,S)-**10** (cf. Fig. 37A / Fig. 37B / Fig. 37C):
   **Yield:** 75 mg (87 %, 0.27 mmol scale)
   **R.f.** 0.10 (CH₂Cl₂/MeOH 10/1, silica gel)
   Free amine: non crystalline solid
   Salt of (*S,S*)-**10** and HCl
   **¹H NMR** (600 MHz, MeOD): δ/ppm = 1.38 - 1.57 (m, 3H), 1.68 - 1.89 (m, 4H), 1.97 - 1.99 (m, 3H), 2.06 - 2.08 (m, 1H), 2.18 - 2.21 (m, 2H), 2.26 - 2.31 (m, 4H), 3.40 (d, *J* = 10.8 Hz, 1H), 3.44 (s, 1H), 3.52 - 3.56 (m, 1H), 3.65 (d, J = 10.8 Hz, 1H), 3.66 - 3.70 (m, 1H), 4.05 (d, *J* = 16.4 Hz, 1 H), 4.15 (d, *J* = 16.4 Hz, 1 H), 4.84 - 4.86 (m, 1 H).
Free amine:
   **¹H NMR** (400 MHz, CH₂Cl₂): δ/ppm = 1.20 (d, *J* = 11.7 Hz, 1 H), 1.32 (d, *J* = 12.5 Hz, 1 H), 1.43 - 1.77 (m, 6H), 1.78 -1.95 (m, 6H), 2.11 - 2.27 (m, 4H), 2.55 - 2.63 (m, 1 H), 3.21 - 3.68 (m, 7H), 4.70 - 4.72 (m, 1 H).
Salt of (*S,S*)-**10** and HCl
   **¹³C NMR** (151 MHz, MeOD): δ/ppm = 26.0 (CH₂), 28.2 (CH), 28.4 (CH), 30.2 (CH), 30.5 (CH₂), 31.1 (CH₂), 34.2 (CH₂), 37.2 (C), 37.3 (CH₂), 37.9 (CH₂), 40.3 (CH₂), 46.9 (CH₂), 47.9 (CH₂), 48.0 (CH), 69.5 (CH), 72.5 (CH₂), 119.2 (C), 165.9 (C).
Free amine:
   **¹³C NMR** (101 MHz, CH₂Cl₂): δ/ppm = 25.6 (CH₂), 28.1 (CH), 28.5 (CH), 30.4 (CH2), 30.5 (CH), 31.2 (CH₂), 34.3 (CH₂), 37.5 (CH₂), 37.7 (C), 38.2 (CH₂), 41.1 (CH₂), 45.9 (CH₂), 47.0(CH), 49.4 (CH₂), 64.9 (CH), 73.2 (CH₂), 118.9 (C), 171.6 (C).
**IR** (neat): ν̃/cm⁻¹ = 3385, 3054, 2905, 2678, 1655, 1412, 1320, 1266, 1192, 1152, 1042, 998, 909, 833, 735, 513.
**HRMS:** m/z = 317.20940 (calculated for C₁₈H₂₇N₃O₂ m/z = 317.21033)

### Crystallographic data

### Substance 2a:

The X-ray crystallographic data of 2a were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. The PLATON MULABS semi-empirical absorption correction using multiple scanned reflection was applied on the data and the structure was solved by direct methods using SHELXS97 and refined with SHELXL2012. All non-hydrogen atoms were refined anisotropically and C-H hydrogen atoms were positioned geometrically. The N-H hydrogen atom was located in difference map and refined isotropic.

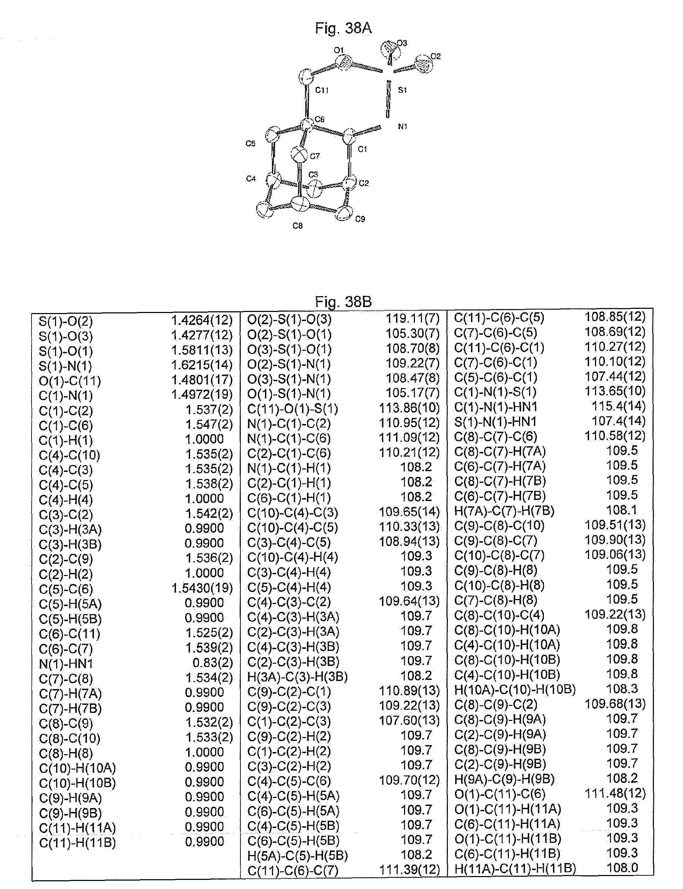

**Crystal data and structure refinement for 2a (cf. Fig. 38A / Fig. 38B):**

| | | |
|---|---|---|
| Empirical formula | C₁₁H₁₇NO₃S | |
| Formula weight | 243.31 | |
| Temperature | 150(2) K | |
| Wavelength | 0.71073 Å | |
| Crystal system | Triclinic | |
| Space group | P-1 | |
| Unit cell dimensions | a = 6.8034(14) Å | a= 89.01(3)°. |
| | b = 8.4147(17) Å | b= 82.10(3)°. |
| | c = 9.957(2) Å | g = 83.89(3)°. |
| Volume | 561.4(2) Å³ | |
| Z | 2 | |
| Density (calculated) | 1.439 Mg/m³ | |
| Absorption coefficient | 0.280 mm⁻¹ | |
| F(000) | 260 | |
| Crystal size | 0.330 x 0.230 x 0.070 mm³ | |
| Theta range for data collection | 2.065 to 27.578°. | |
| Index ranges | -8<=h<=8, -10<=k<=10, -12<=I<=12 | |
| Reflections collected | 20027 | |
| Independent reflections | 2572 [R(int) = 0.0613] | |
| Completeness to theta = 25.242° | 100.0 % | |
| Absorption correction | Empirical | |
| Max. and min. transmission | 0.7452 and 0.6752 | |
| Refinement method | Full-matrix least-squares on F² | |
| Data / restraints / parameters | 2572 / 0 / 149 | |
| Goodness-of-fit on F² | 1.082 | |
| Final R indices [I>2sigma(I)] | R1 = 0.0359, wR2 = 0.0992 | |
| R indices (all data) | R1 = 0.0471, wR2 = 0.1036 | |
| Extinction coefficient | n/a | |
| Largest diff. peak and hole | 0.233 and -0.441 e.Å⁻³ | |

### Substance 2b:

The X-ray crystallographic data of **2b** were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. The PLATON MULABS semi-empirical absorption correction using multiple scanned reflection was applied on the data and the structure was solved by direct methods using SHELXS97 and refined with SHELXL2014. All non-hydrogen atoms were refined
anisotropically and C-H hydrogen atoms were positioned geometrically. The N-H hydrogen atoms were located in difference map and refined isotropic with its bond lengths set to ideal values.

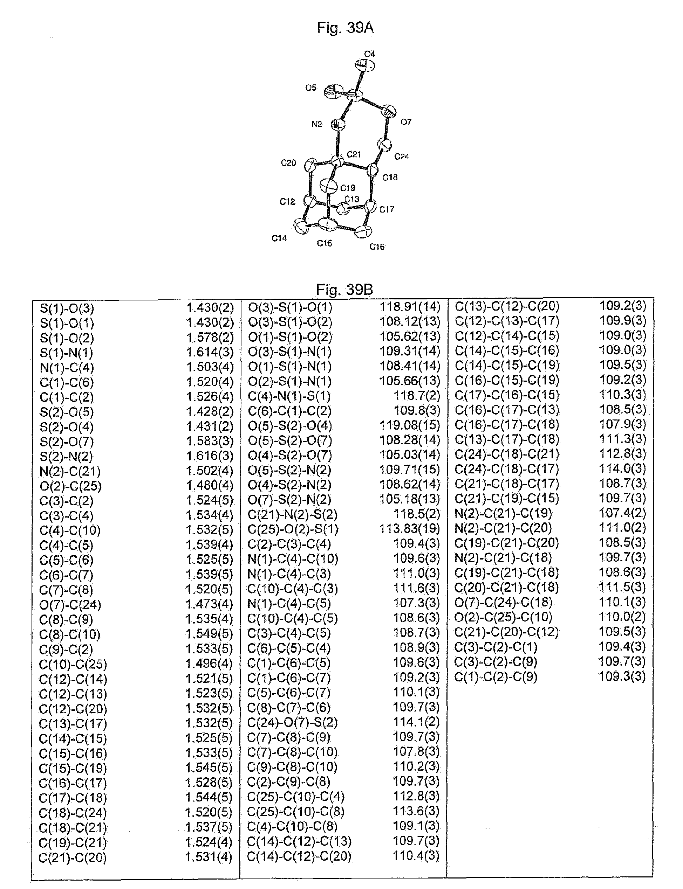

**Crystal data and structure refinement for 2b (cf. Fig. 39A / Fig. 39B):**

| | | |
|---|---|---|
| Empirical formula | C₂₂H₃₄N₂O₆S₂ | |
| Formula weight | 486.63 | |
| Temperature | 150(2) K | |
| Wavelength | 0.71073 Å | |
| Crystal system | Orthorhombic | |
| Space group | Pbca | |
| Unit cell dimensions | a = 13.173(3) Å | a= 90°. |
| | b = 13.173(3) Å | b= 90°. |
| | c = 25.804(5) Å | g = 90°. |
| Volume | 4477.7(15) Å³ | |
| Z | 8 | |
| Density (calculated) | 1.444 Mg/m³ | |
| Absorption coefficient | 0.281 mm⁻¹ | |
| F(000) | 2080 | |
| Crystal size | 0.380 x 0.160 x 0.100 mm³ | |
| Theta range for data collection | 1.578 to 25.019°. | |
| Index ranges | -13<=h<=13, -10<=k<=15, -30<=I<=26 | |
| Reflections collected | 12568 | |
| Independent reflections | 3700 [R(int) = 0.1023] | |
| Completeness to theta = 25.019° | 93.5 % | |
| Absorption correction | Empirical | |
| Max. and min. transmission | 1.11309 and 0.88601 | |
| Refinement method | Full-matrix least-squares on F² | |
| Data / restraints / parameters | 3700 / 2 / 297 | |
| Goodness-of-fit on F² | 1.025 | |
| Final R indices [I>2sigma(I)] | R1 = 0.0501, wR2 = 0.1151 | |
| R indices (all data) | R1 = 0.0886, wR2 = 0.1408 | |
| Extinction coefficient | n/a | |
| Largest diff. peak and hole | 0.374 and -0.574 e.Å⁻³ | |

### Substance 2d:

For data collection of **2d** a BRUKER D8 Venture system equipped with dual IµS microfocus sources, a PHOTON100 detector and an OXFORD CRYOSYSTEMS 700 low temperature system was used. Data collection was performed using Mo-K_{α} radiation with wavelength 0.71073 Å and a collimating Quazar multilayer mirror. Semi-empirical absorption correction from equivalents was applied using SADABS-2014/4 and the structure was solved by intrinsic phasing using SHELXT2014. Refinement was performed against F² using SHELXL-2014. All non-hydrogen atoms were refined anisotropically and C-H hydrogen atoms were positioned geometrically. The N-H hydrogen atom was located in difference map and refined isotropic.

**Crystal data and structure refinement for 2d (cf. Fig. 40A / Fig. 40B):**

| | | |
|---|---|---|
| Empirical formula | C₁₁H₁₆Br NO₃S | |
| Formula weight | 322.22 | |
| Temperature | 100(2) K | |
| Wavelength | 0.71073 Å | |
| Crystal system | Monoclinic | |
| Space group | P2₁/n | |
| Unit cell dimensions | a = 8.7124(3) Å | a= 90°. |
| | b = 11.1753(4) Å | b= 101.854(2)°. |
| | c = 13.0091(5) Å | g = 90°. |
| Volume | 1239.60(8) Å³ | |
| Z | 4 | |
| Density (calculated) | 1.727 Mg/m³ | |
| Absorption coefficient | 3.480 mm⁻¹ | |
| F(000) | 656 | |
| Crystal size | 0.228 x 0.222 x 0.164 mm³ | |
| Theta range for data collection | 2.425 to 25.026°. | |
| Index ranges | -10<=h<=10, -13<=k<=13, -4<=I<=15 | |
| Reflections collected | 2195 | |
| Independent reflections | 2195 [R(int) = ?] | |
| Completeness to theta = 25.026° | 100.0 % | |
| Absorption correction | Semi-empirical from equivalents | |
| Max. and min. transmission | 0.7461 and 0.5964 | |
| Refinement method | Full-matrix least-squares on F² | |
| Data / restraints / parameters | 2195 / 0 / 159 | |
| Goodness-of-fit on F² | 1.137 | |
| Final R indices [I>2sigma(I)] | R1 = 0.0225, wR2 = 0.0541 | |
| R indices (all data) | R1 = 0.0241, wR2 = 0.0548 | |
| Extinction coefficient | n/a | |
| Largest diff. peak and hole | 0.403 and -0.448 e.Å⁻³ | |

### Substance 2e:

The X-ray crystallographic data of **2e** were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. The PLATON MULABS semi-empirical absorption correction using multiple scanned reflection was applied on the data and the structure was solved by direct methods using SHELXS97 and refined with SHELXL2014. All non-hydrogen atoms were refined
anisotropically and C-H hydrogen atoms were positioned geometrically. The N-H hydrogen atom was located in difference map and refined isotropic.

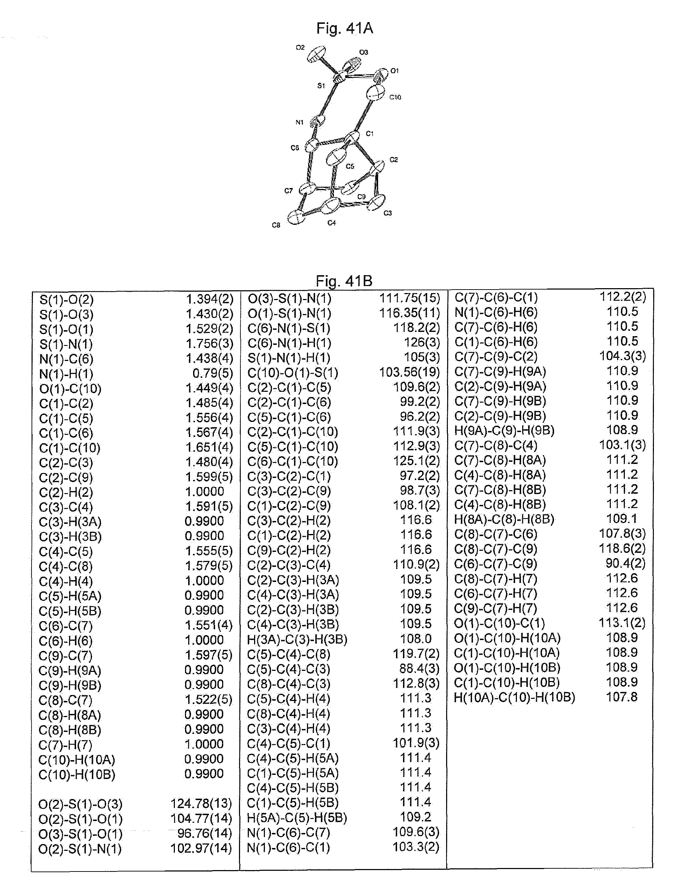

**Crystal data and structure refinement for 2e (cf. Fig. 41A / Fig. 41 B):**

| | | |
|---|---|---|
| Empirical formula | C₁₀H₁₅NO₃S | |
| Formula weight | 229.29 | |
| Temperature | 150(2) K | |
| Wavelength | 0.71073 Å | |
| Crystal system | Orthorhombic | |
| Space group | P2₁2₁2₁ | |
| Unit cell dimensions | a = 9.3037(19) Å | a= 90°. |
| | b = 9.846(2) Å | b= 90°. |
| | c = 11.183(2) Å | g = 90°. |
| Volume | 1024.4(4) Å³ | |
| Z | 4 | |
| Density (calculated) | 1.487 Mg/m³ | |
| Absorption coefficient | 0.302 mm⁻¹ | |
| F(000) | 488 | |
| Crystal size | 0.300 x 0.200 x 0.200 mm³ | |
| Theta range for data collection | 2.756 to 31.272°. | |
| Index ranges | -12<=h<=10, -14<=k<=14, -12<=I<=12 | |
| Reflections collected | 16235 | |
| Independent reflections | 2347 [R(int) = 0.0614] | |
| Completeness to theta = 25.242° | 98.8 % | |
| Absorption correction | Empirical | |
| Max. and min. transmission | 0.93145 and 0.85917 | |
| Refinement method | Full-matrix least-squares on F² | |
| Data / restraints / parameters | 2347 / 0 / 141 | |
| Goodness-of-fit on F² | 1.089 | |
| Final R indices [I>2sigma(I)] | R1 = 0.0400, wR2 = 0.1053 | |
| R indices (all data) | R1 = 0.0464, wR2 = 0.1075 | |
| Absolute structure parameter | 0.19(13) | |
| Extinction coefficient | n/a | |
| Largest diff. peak and hole | 0.320 and -0.470 e.Å⁻³ | |

### Substance 2f:

The X-ray crystallographic data of **2f** were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. A semi-empirical absorption correction using multiple scanned reflection was applied on the data using SADABS-2008/1 and the structure was solved by intrinsic phasing using SHELXT2014 and refined with SHELXL2014. All non-hydrogen atoms were refined
anisotropically and C-H hydrogen atoms were positioned geometrically. The N-H hydrogen atom was located in difference map and refined isotropic.

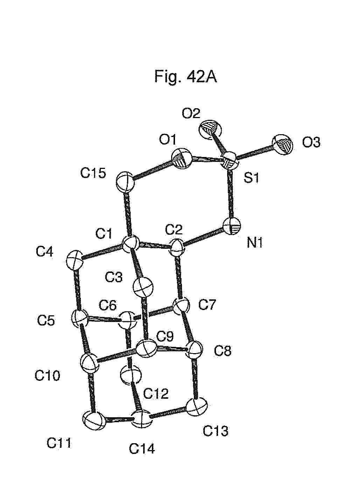

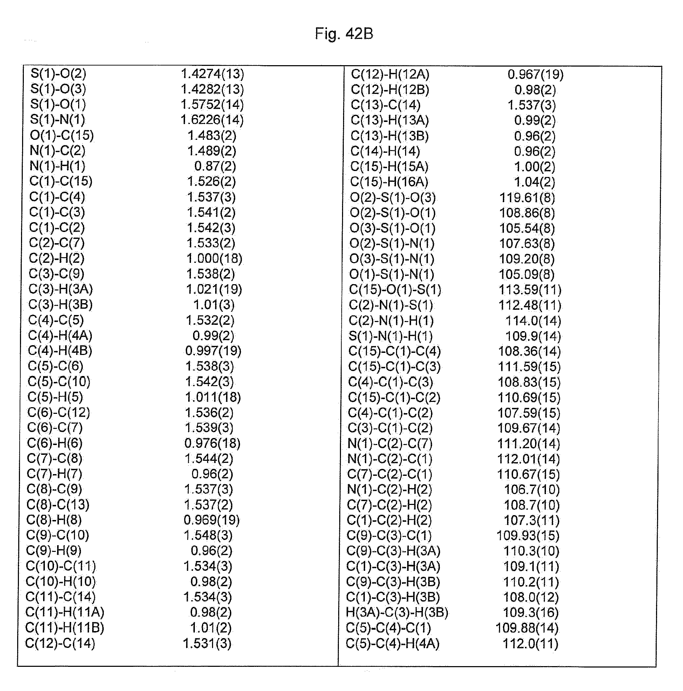

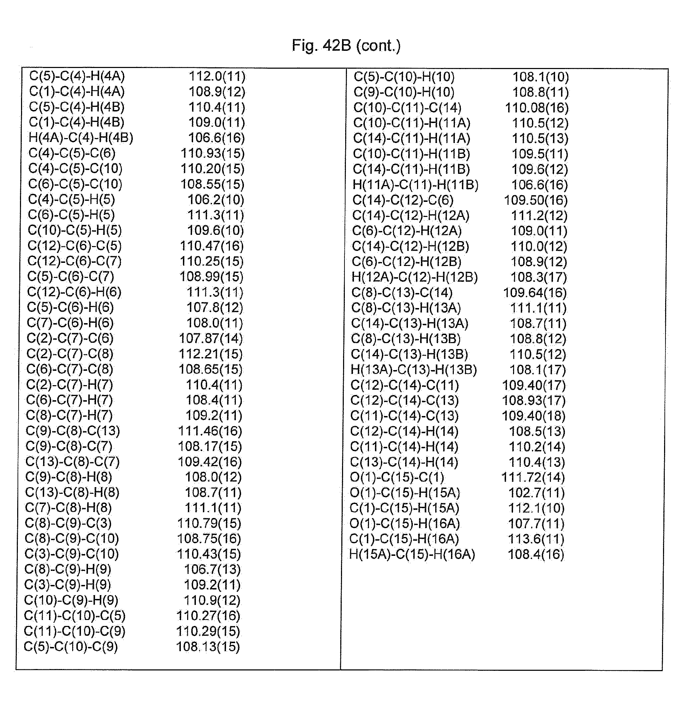

**Crystal data and structure refinement for 2f (cf. Fig. 42A / Fig. 42B):**

| | | |
|---|---|---|
| Empirical formula | C₁₅H₂₁NO₃S | |
| Formula weight | 295.39 | |
| Temperature | 150(2) K | |
| Wavelength | 0.71073 Å | |
| Crystal system | Monoclinic | |
| Space group | P2₁/c | |
| Unit cell dimensions | a = 9.4965(19) Å | a= 90°. |
| | b = 11.494(2) Å | b= 103.80(3)°. |
| | c = 12.720(3) Å | g = 90°. |
| Volume | 1348.3(5) Å³ | |
| Z | 4 | |
| Density (calculated) | 1.455 Mg/m³ | |
| Absorption coefficient | 0.248 mm⁻¹ | |
| F(000) | 632 | |
| Crystal size | 0.110 x 0.100 x 0.100 mm³ | |
| Theta range for data collection | 2.208 to 31.045°. | |
| Index ranges | -13<=h<=13, -14<=k<=16, -18<=I<=15 | |
| Reflections collected | 20980 | |
| Independent reflections | 4303 [R(int) = 0.1160] | |
| Completeness to theta = 25.242° | 100.0 % | |
| Absorption correction | Empirical | |
| Max. and min. transmission | 0.7462 and 0.5881 | |
| Refinement method | Full-matrix least-squares on F² | |
| Data / restraints / parameters | 4303 / 0 / 265 | |
| Goodness-of-fit on F² | 0.952 | |
| Final R indices [I>2sigma(I)] | R1 = 0.0484, wR2 = 0.1013 | |
| R indices (all data) | R1 = 0.1004, wR2 = 0.1179 | |
| Extinction coefficient | n/a | |
| Largest diff. peak and hole | 0.282 and -0.441 e.Å⁻³ | |

### Substance 2g:

The X-ray crystallographic data of **2g** were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. A semi-empirical absorption correction using multiple scanned reflection was applied on the data using SADABS-2008/1 and the structure was solved by direct methods using SHELXS97 and refined with SHELXL2014. All non-hydrogen atoms were refined
anisotropically and hydrogen atoms were positioned geometrically.

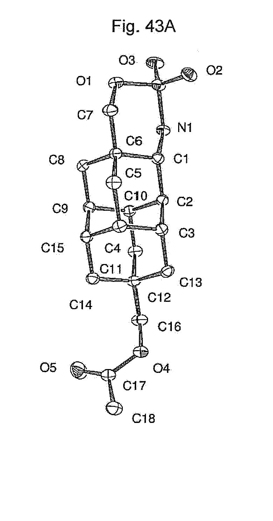

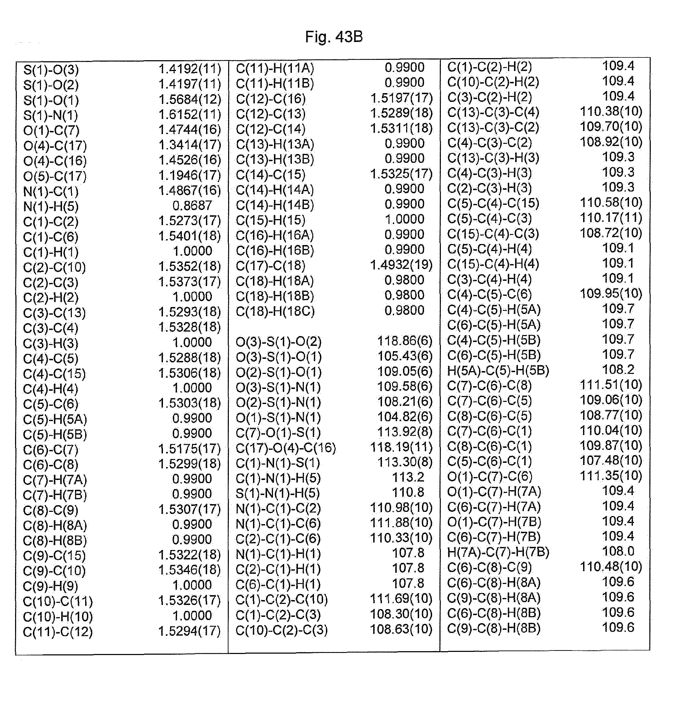

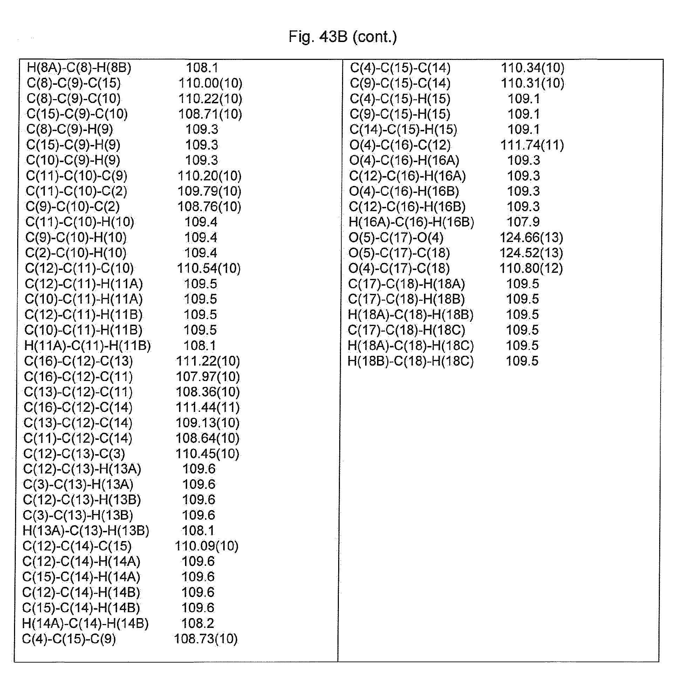

**Crystal data and structure refinement for 2g (cf. Fig. 43A / Fig. 43B):**

| | | |
|---|---|---|
| Empirical formula | C₁₈H₂₅NO₅S | |
| Formula weight | 367.45 | |
| Temperature | 150(2) K | |
| Wavelength | 0.71073 Å | |
| Crystal system | Triclinic | |
| Space group | P-1 | |
| Unit cell dimensions | a = 8.3212(17) Å | a= 88.63(3)°. |
| | b = 8.3560(15) Å | b= 80.02(3)°. |
| | c = 11.934(2) Å | g = 82.72(3)°. |
| Volume | 810.6(3) Å³ | |
| Z | 2 | |
| Density (calculated) | 1.505 Mg/m³ | |
| Absorption coefficient | 0.231 mm⁻¹ | |
| F(000) | 392 | |
| Crystal size | 0.300 x 0.270 x 0.250 mm³ | |
| Theta range for data collection | 1.733 to 31.491°. | |
| Index ranges | -12<=h<=12, -12<=k<=11, -17<=I<=17 | |
| Reflections collected | 23476 | |
| Independent reflections | 5249 [R(int) = 0.0515] | |
| Completeness to theta = 25.242° | 99.9 % | |
| Absorption correction | Empirical | |
| Max. and min. transmission | 0.7208 and 0.6491 | |
| Refinement method | Full-matrix least-squares on F² | |
| Data / restraints / parameters | 5249 / 0 / 227 | |
| Goodness-of-fit on F² | 1.051 | |
| Final R indices [I>2sigma(I)] | R1 = 0.0423, wR2 = 0.1148 | |
| R indices (all data) | R1 = 0.0577, wR2 = 0.1231 | |
| Extinction coefficient | n/a | |
| Largest diff. peak and hole | 0.348 and -0.442 e.Å⁻³ | |

### Substance 2h:

The X-ray crystallographic data of **2h** were collected using a BRUKER/NONIUS KappaCCD detector with a BRUKER/NONIUS FR591 rotating anode radiation source and an OXFORD CRYOSYSTEMS 600 low temperature system. Mo-K_{α} radiation with wavelength 0.71073 Å and a graphite monochromator were used. The PLATON MULABS semi-empirical absorption correction using multiple scanned reflection was applied on the data and the structure was solved by direct methods using SHELXS97 and refined with SHELXL2014. All non-hydrogen atoms were refined
anisotropically and C-H hydrogen atoms were positioned geometrically. The N-H hydrogen atoms were located in difference map and refined isotropic.

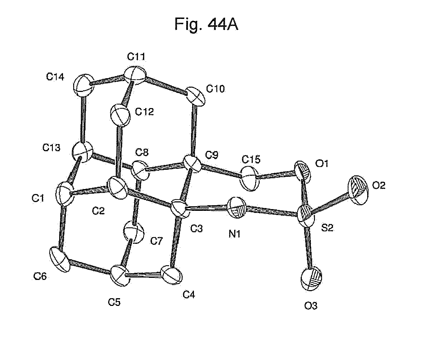

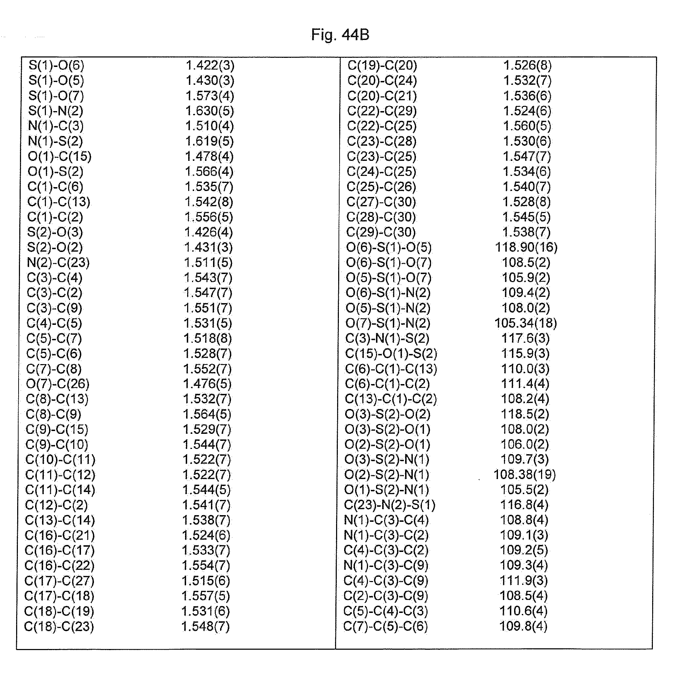

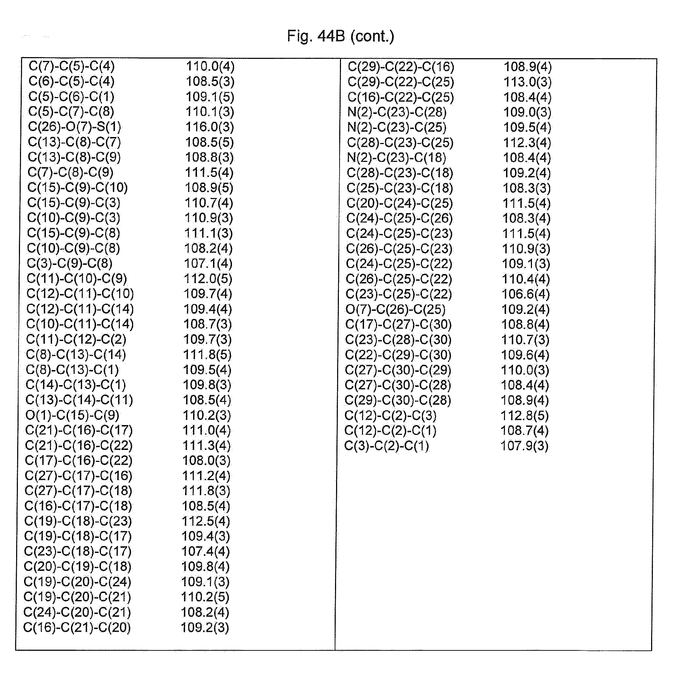

**Crystal data and structure refinement for 2h (cf. Fig. 44A / Fig. 44B):**

| | | |
|---|---|---|
| Empirical formula | C15 H21 N O3 S | |
| Formula weight | 295.39 | |
| Temperature | 150(2) K | |
| Wavelength | 0.71073 Å | |
| Crystal system | Triclinic | |
| Space group | P-1 | |
| Unit cell dimensions | a = 6.5990(13) Å | a= 90.10(3)°. |
| | b = 13.143(3) Å | b= 92.43(3)°. |
| | c = 15.356(3) Å | g = 98.78(3)°. |
| Volume | 1315.0(5) Å³ | |
| Z | 4 | |
| Density (calculated) | 1.492 Mg/m³ | |
| Absorption coefficient | 0.254 mm⁻¹ | |
| F(000) | 632 | |
| Crystal size | 0.420 x 0.290 x 0.140 mm³ | |
| Theta range for data collection | 1.327 to 27.556°. | |
| Index ranges | -8<=h<=7, -17<=k<=17, -18<=I<=19 | |
| Reflections collected | 8265 | |
| Independent reflections | 8265 [R(int) = ?] | |
| Completeness to theta = 25.242° | 64.4 % | |
| Absorption correction | Empirical | |
| Max. and min. transmission | 1.13673 and 0.82180 | |
| Refinement method | Full-matrix least-squares on F² | |
| Data / restraints / parameters | 8265 / 0 / 370 | |
| Goodness-of-fit on F² | 1.065 | |
| Final R indices [I>2sigma(I)] | R1 = 0.0655, wR2 = 0.1870 | |
| R indices (all data) | R1 = 0.0868, wR2 = 0.1992 | |
| Extinction coefficient | n/a | |
| Largest diff. peak and hole | 0.340 and -0.461 e.Å⁻³ | |

### (R)-6 with (R)-mandelic acid:

For data collection of **(*R*)-6 with (*R*)-mandelic** acid a BRUKER D8 Venture system equipped with dual IµS microfocus sources, a PHOTON100 detector and an OXFORD CRYOSYSTEMS 700 low temperature system was used. Data collection was performed using Cu-K_{α} radiation with wavelength 1.54178 Å and a collimating Quazar multilayer mirror. Semi-empirical absorption correction from equivalents was applied using SADABS-2014/4 and the structure was solved by intrinsic phasing using SHELXT2014. Refinement was performed against F² using SHELXL-2014. All non-hydrogen atoms were refined anisotropically and C-H hydrogen atoms were positioned geometrically. The N-H and O-H hydrogen atoms were located in difference map, the O-H distance was set to ideal values and both were refined isotropic.

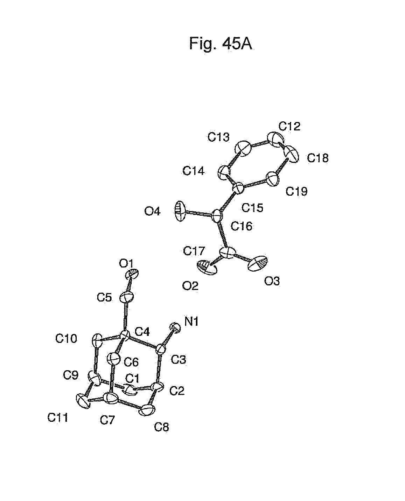

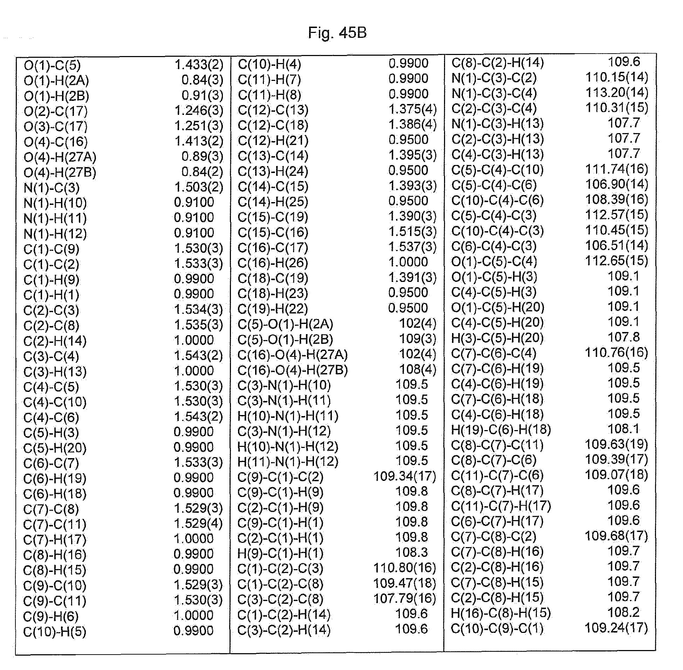

**Crystal data and structure refinement for (R)-6 with (R)-mandelic acid (cf. Fig. 45A / Fig. 45B):**

| | | |
|---|---|---|
| Empirical formula | C₁₉H₂₇NO₄ | |
| Formula weight | 333.41 | |
| Temperature | 100(2) K | |
| Wavelength | 1.54178 Å | |
| Crystal system | Monoclinic | |
| Space group | C2 | |
| Unit cell dimensions | a = 21.1694(18) Å | a= 90°. |
| | b = 6.5741(6) Å | b= 127.536(2)°. |
| | c = 16.0992(14) Å | g = 90°. |
| Volume | 1776.7(3) Å³ | |
| Z | 4 | |
| Density (calculated) | 1.246 Mg/m³ | |
| Absorption coefficient | 0.701 mm⁻¹ | |
| F(000) | 720 | |
| Crystal size | 0.509 x 0.285 x 0.047 mm³ | |
| Theta range for data collection | 4.184 to 74.757°. | |
| Index ranges | -26<=h<=26, -7<=k<=8, -20<=I<=19 | |
| Reflections collected | 15589 | |
| Independent reflections | 3521 [R(int) = 0.0532] | |
| Completeness to theta = 67.679° | 99.8 % | |
| Absorption correction | Semi-empirical from equivalents | |
| Max. and min. transmission | 0.7538 and 0.5354 | |
| Refinement method | Full-matrix least-squares on F² | |
| Data / restraints / parameters | 3521 / 5 / 233 | |
| Goodness-of-fit on F² | 1.078 | |
| Final R indices [I>2sigma(I)] | R1 = 0.0326, wR2 = 0.0835 | |
| R indices (all data) | R1 = 0.0341, wR2 = 0.0849 | |
| Absolute structure parameter | 0.2(2) | |
| Extinction coefficient | 0.0039(4) | |
| Largest diff. peak and hole | 0.216 and -0.153 e.Å⁻³ | |

### (R,S)-10 as a salt with (R)-mandelic acid:

For data collection of **(*R*,*S*)-10 as a salt with (*R*)-mandelic acid a** BRUKER D8 Venture system equipped with dual IµS microfocus sources, a PHOTON100 detector and an OXFORD CRYOSYSTEMS 700 low temperature system was used. Data collection was performed using Cu-K_{α} radiation with wavelength 1.54178 Å and a collimating Quazar multilayer mirror. Semi-empirical absorption correction from equivalents was applied using SADABS-2014/4 and the structure was solved by intrinsic phasing using SHELXT2014. Refinement was performed against F² using SHELXL-2014. All non-hydrogen atoms were refined anisotropically and hydrogen atoms were positioned geometrically. The O3-H hydrogen atom was located in difference map and refined isotropic.

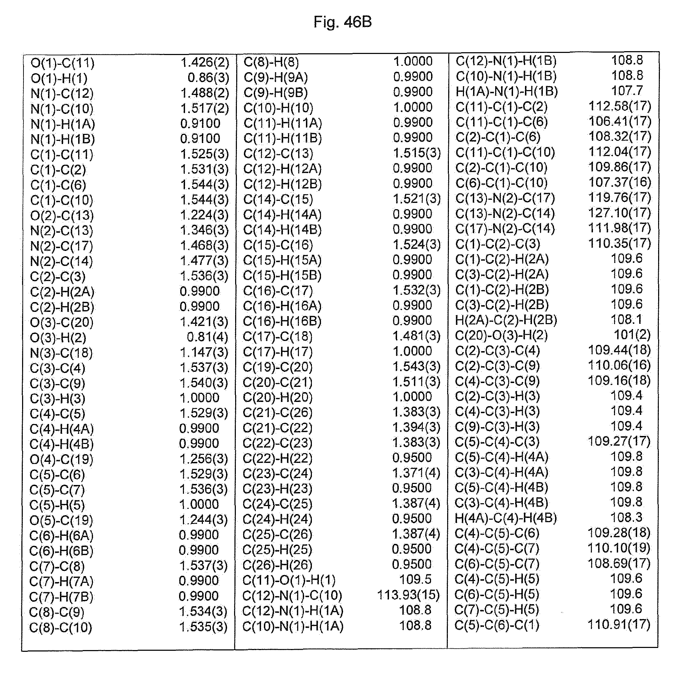

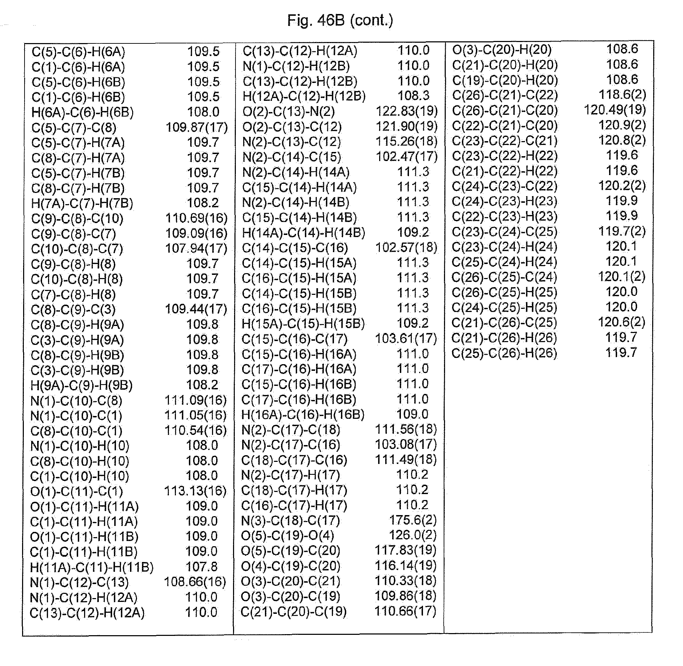

**Crystal data and structure refinement for (R,S)-10 as a salt with (R)-mandelic acid (cf. Fig. 46A / Fig. 46B):**

| | | |
|---|---|---|
| Empirical formula | C₂₆H₃₅N₃O₅ | |
| Formula weight | 469.57 | |
| Temperature | 100(2) K | |
| Wavelength | 1.54178 Å | |
| Crystal system | Orthorhombic | |
| Space group | P2₁2₁2₁ | |
| Unit cell dimensions | a = 7.23120(10) Å | a= 90°. |
| | b = 9.0912(2) Å | b= 90°. |
| | c = 36.4500(7) Å | g = 90°. |
| Volume | 2396.23(8) Å³ | |
| Z | 4 | |
| Density (calculated) | 1.302 Mg/m³ | |
| Absorption coefficient | 0.734 mm⁻¹ | |
| F(000) | 1008 | |
| Crystal size | 0.438 x 0.344 x 0.280 mm³ | |
| Theta range for data collection | 2.424 to 67.515°. | |
| Index ranges | -7<=h<=8, -10<=k<=10, -42<=I<=43 | |
| Reflections collected | 16900 | |
| Independent reflections | 4311 [R(int) = 0.0403] | |
| Completeness to theta = 67.679° | 99.8 % | |
| Absorption correction | Semi-empirical from equivalents | |
| Max. and min. transmission | 0.7530 and 0.5870 | |
| Refinement method | Full-matrix least-squares on F² | |
| Data / restraints / parameters | 4311 / 0 / 314 | |
| Goodness-of-fit on F² | 1.092 | |
| Final R indices [I>2sigma(I)] | R1 = 0.0326, wR2 = 0.0765 | |
| R indices (all data) | R1 = 0.0355, wR2 = 0.0779 | |
| Absolute structure parameter | 0.1(2) | |
| Extinction coefficient | n/a | |
| Largest diff. peak and hole | 0.160 and -0.243 e.Å⁻³ | |

## Claims

1. Method for synthesizing 1,2-disubstituted diamondoids **characterized in that** the method comprises the following steps:
1. esterification of hydroxymethyl diamondoid by use of a substance chosen from the list comprising R₆-SO₂N₃, R₆-SONH₂, R₆-SO₂NHR₇, R₆-SONHR₇, where R₆ and R₇ are chosen from the list, comprising halogenid-, tosyl-, mesyl- and CF₃COO-,
2. performing on the product of step 1 a transition metal catalysed intramolecular oxidative insertion reaction of the functional group introduced via step 1 into a neighboring CH-bond of the diamondoid core structure.

2. Method for synthesizing 1,2-disubstituted diamondoids according to claim 1, **characterized in that** the product of step 2 from claim 1 is processed by the following steps:
3. performing on the product of step 2 a reductive cleavage of the cyclic sulfamate N-S bond and/or O-S bond by usage of a reducing agent chosen from the list comprising NMeEt₂*AlH₃ complex, AlH₃, LiAlH₄ and NaH, leading to a racemic mixture of products,
4. separating the racemic mixture of products from step 3 into the enantiomeric components via salt formation with enantiopure mandelic acid and recrystallisation the mixture of salts,
5. releasing the free enantiopure 1,3-amino-alcohols from the products of step 4 by use of organic or inorganic base chosen from the list comprising carbonates, hydroxides, hydrogencarbonates, and
6. condensation of each of the enantiopure products of step 5 with pyrrolidine derivate 9.

3. Method for synthesizing 1,2-disubstituted diamondoids according to claim 1, **characterized in that** the product of step 2 from claim 1 is processed by the following steps:
7. oxidizing the product of step 2 by use of alkaline aqueous permanganate solution and
8. further functionalizing the product from step 7 by usage of a method chosen from the list comprising the methods hydrolysation with Broensted acids and nucleophilic addition with Grignard-reagent.

4. Substances produced by the method according to claim 1, **characterized by** the general formula (IV), (V) and (VI), 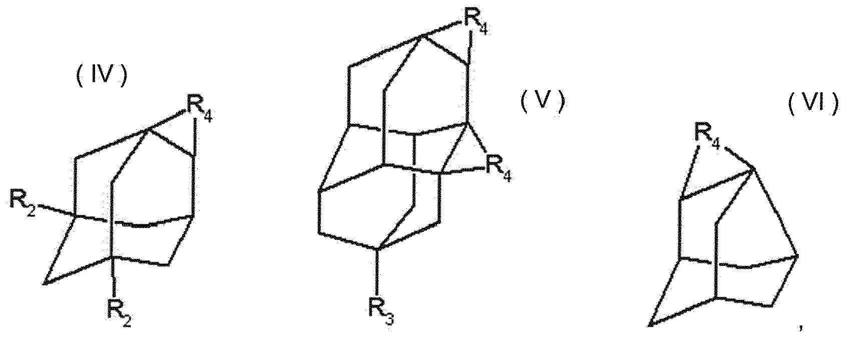 **characterized in that**
a. R₂ is chosen from the list comprising -H, -CH₃, -CH₂CH₃, -Br, -Cl, -I;
b. R₃ is chosen from the list comprising -H, CH₃-CO₂-CH₂-, C₂H₅-CO₂-CH₂-C₃H₇-CO₂-CH₂- and C₄H₉-CO₂-CH₂-;
c. R₄ is chosen from the list comprising -CH₂-OSO₂NH- and -CH₂-OSONH-.

5. Substances produced by the method according to claim 2, **characterized by** the general formula (I), (II) and (III), 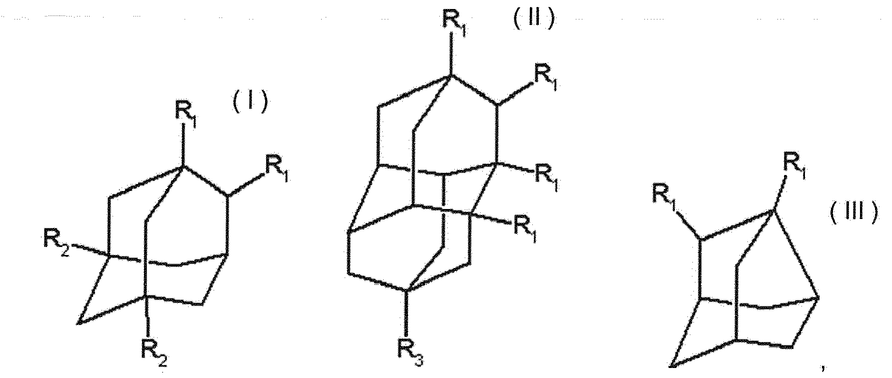 **characterized in that**
a. R₁ is chosen from the list comprising -H, -CH₂-OSO₂NH₂, -CH₂-OSO₂N₃,-CH₂-OSONH₂, -CH₂-OSO₂NHR, -CH₂-OSONHR, and R is chosen from the list comprising halogenid-, tosyl-, mesyl-, CF₃COO-;
b. R₂ is chosen from the list comprising -H, -CH₃, -CH₂CH₃, -Br, -Cl, -I;
c. R₃ is chosen from the list comprising -H, CH₃-CO₂-CH₂-, C₂H₅-CO₂-CH₂-, C₃H₇-CO₂-CH₂- and C₄H₉-CO₂-CH₂-;
in such a way that one of two R₁-substiuents at neighboring C-atom positions of the cage structures consists of just one H-atom and the second R₁-substituent is any other one substituent of the R₁-Substituents.

6. Substances produced by the method according to claim 3, **characterized by** the general formula (VII), 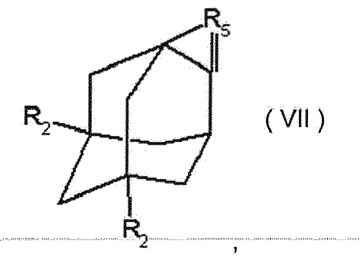 **characterized in that**
a. R₂ is chosen from the list comprising -H, -CH₃, -CH₂CH₃, -Br, -Cl, -I;
b. R₅ is chosen from the list comprising -CH₂-OSO₂N=, -CH₂-OSON=.

7. Substances according to claims 4 to 6, **characterized in that** they are deprotonated by use of an organic or inorganic base and thus are available as salts.

8. Substances according to claim 4 to 6, **characterized in that** they are protonated by use of an organic or inorganic acid and thus are available as salts.

9. Use of substances according to claims 4 to 8 as pharmaceutical component for medical treatment of mammalian diseases, **characterized in that** the disease to be treated is chosen from the list comprising autoimmune diseases, neurodegenerative diseases, diseases in which cells are destroyed by cytopathic effects, infections of viruses, infections of retroviruses and borna disease virus infections.

10. Use of at least one substance according to claims 4 to 8 as monomer and/or comonomer for polymerization.

11. Use of at least one substance according to claims 4 to 8 as polymer-additive.

12. Use of at least one substance according to claims 4 to 8 as polymer-additive for crosslinking polymers.

13. Use of at least one substance according to claims 4 to 8 for functionalizing surfaces of macroscopic, microscopic or nanoscale objects.

14. Use of at least one substance according to claims 4 to 8 as intermediate in the synthesis of bioactive compounds, ligands for metals, organocatalysts, surfactants, isolants, and macrocycles.
